# FASCICULE DE BREVET EUROPEEN

(11) **EP 1 704 230 B1**
(45) Date de publication et mention de la délivrance du brevet: **19.08.2009**
(21) Numéro de dépôt: 05717405.4
(22) Date de dépôt: 12.01.2005
(51) Int. Cl.: C12N 15/01, C12N 9/02, C12N 1/21, C12P 7/18, C12P 7/28

(54) **MICROORGANISME EVOLUE POUR LA PRODUCTION DE 1,2-PROPANEDIOL**
FORTGESCHRITTENER MIKROORGANISMUS ZUR PRODUKTION VON 1,2-PROPANDIOL
ADVANCED MICROORGANISM FOR PRODUCING 1,2-PROPANEDIOL

(30) Priorité: 12.01.2004 FR 0400214
(43) Date de publication de la demande: 27.09.2006
(73) Titulaire: Metabolic Explorer, 63360 Saint Beauzire (FR)
(72) Inventeur: MEYNIAL-SALLES, Isabelle, F-31450 Fourquevaux (FR); GONZALEZ, Benjamin, F-63200 Riom (FR); SOUCAILLE, Philippe, F-31450 Deyme (FR)
(74) Mandataire: Tetaz, Franck Claude Edouard
(86) Numéro de dépôt international: PCT/FR2005/000070
(87) Numéro de publication internationale: WO 2005/073364

(56) Documents cités:
- WO-A-98/37204
- ALTARAS NEDIM E ET AL: "Metabolic engineering of a 1,2-Propanediol pathway in Escherichia coli" APPLIED AND ENVIRONMENTAL MICROBIOLOGY, vol. 65, no. 3, mars 1999 (1999-03), pages 1180-1185, XP002293970 ISSN: 0099-2240 cité dans la demande
- ALTARAS NEDIM E ET AL: "Enhanced production of (R)-1,2-propanediol by metabolically engineered Escherichia coli" BIOTECHNOLOGY PROGRESS, vol. 16, no. 6, novembre 2000 (2000-11), pages 940-946, XP002293971 ISSN: 8756-7938 cité dans la demande
- GIRBAL L ET AL: "Regulation of solvent production in Clostridium acetobutylicum" TRENDS IN BIOTECHNOLOGY, ELSEVIER PUBLICATIONS, CAMBRIDGE, GB, vol. 16, no. 1, 1998, pages 11-16, XP004101891 ISSN: 0167-7799
- CAMERON D C ET AL: "METABOLIC ENGINEERING OF PROPANEDIOL PATHWAYS" BIOTECHNOLOGY PROGRESS, XX, XX, vol. 14, no. 1, 6 février 1998 (1998-02-06), pages 116-125, XP002067772 ISSN: 8756-7938 cité dans la demande
- TRAN-DIN K ET AL: "FORMATION OF D(-)-1,2-PROPANEDIOL AND D(-)-LACTATE FROM GLUCOSE BY CLOSTRIDIUM SPHENOIDES UNDER PHOSPHATE LIMITATION" ARCHIVES OF MICROBIOLOGY, BERLIN, DE, vol. 142, 1985, pages 87-92, XP002067777 ISSN: 0302-8933 cité dans la demande
- SNOEP JACKY L ET AL: "Differences in sensitivity to NADH of purified pyruvate dehydrogenase complexes of Enterococcus faecalis, Lactococcus lactis, Azotobacter vinelandii and Escherichia coli: Implications for their activity in vivo" FEMS (FEDERATION OF EUROPEAN MICROBIOLOGICAL SOCIETIES) MICROBIOLOGY LETTERS, vol. 114, no. 3, 1993, pages 279-283, XP002293972 ISSN: 0378-1097 cité dans la demande
- STEPHENS P E ET AL: "NUCLEOTIDE SEQUENCE OF THE LIPOAMIDE DEHYDROGENASE GENE OF ESCHERICHIA COLI K12" EUROPEAN JOURNAL OF BIOCHEMISTRY, BERLIN, DE, vol. 135, no. 3, 1983, pages 519-528, XP008014488 ISSN: 0014-2956 -& DATABASE UniProt [Online] 21 juillet 1986 (1986-07-21), "Dihydrolipoyl dehydrogenase (EC 1.8.1.4) (E3 component of pyruvate and 2-oxoglutarate dehydrogenases complexes) (Dihydrolipoamide dehydrogenase) (Glycine cleavage system L protein)." XP002334993 extrait de EBI accession no. UNIPROT:DLDH_ECOLI Database accession no. P00391

### SEQUENCE LISTING

<110> Metabolic Explorer
<120> Microorganisme Evolué pour la Production de 1,2-propanediol
<130> 347276
<150> FR 0400214
   <151> 2004-01-12
<160> 36
<170> PatentIn version 3.1
<210> 1
   <211> 102
   <212> DNA
   <213> Artificial
<400> 1
   atgcgacatc ctttagtgat gggtaactgg aaactgaacg gcagccgcca catggttcac 60
   gagctggttt ctaacctgcg tacatatgaa tatcctcctt ag 102
<210> 2
   <211> 100
   <212> DNA
   <213> Artificial
<400> 2
   cttaagcctg tttagccgct tctgcagctt taacgattac tgcgaaggcg tcagctttca 60
   gagaagcacc accaaccagc tgtaggctgg agctgcttcg 100
<210> 3
   <211> 20
   <212> DNA
   <213> Artificial
<400> 3
   ggtgatgata gttatcgccg 20
<210> 4
   <211> 20
   <212> DNA
   <213> Artificial
<400> 4
   cgtgccatcg acagcagtcc 20
<210> 5
   <211> 100
   <212> DNA
   <213> Artificial
<400> 5
   ccggacatcc tgcgttgccg taaatctggt gttctgaccg gtctgccaga tgcatatggc 60
   cgtggccgta tcatcggtga catatgaata tcctccttag 100
<210> 6
   <211> 94
   <212> DNA
   <213> Artificial
<400> 6
   gatgcactat aagatgtgtt aaaaacgctg tagcagaatg aagcgcggaa taaaaaagcg 60
   gcaactcaat aaagttgccg ctggagctgc ttcg 94
<210> 7
   <211> 30
   <212> DNA
   <213> Artificial
<400> 7
   agacattaaa aatatacgtg cagctacccg 30
<210> 8
   <211> 30
   <212> DNA
   <213> Artificial
<400> 8
   gtgaaagctg acaacccttt tgatctttta 30
<210> 9
   <211> 101
   <212> DNA
   <213> Artificial
<400> 9
   atggctgtta ctaatgtcgc tgaacttaac gcactcgtag agcgtgtaaa aaaagcccag 60
   cgtgaatatg ccagtttcac tcatatgaat atcctcctta g 101
<210> 10
   <211> 100
   <212> DNA
   <213> Artificial
<400> 10
   caataacgaa tgatagcaat tttaagtagt taggaggtga aaaatgctgt caaaaggcgt 60
   attgtcagcg cgtcttttca tgtaggctgg agctgcttcg 100
<210> 11
   <211> 22
   <212> DNA
   <213> Artificial
<400> 11 22
   ggctcattgc accaccatcc ag 22
<210> 12
   <211> 24
   <212> DNA
   <213> Artificial
<400> 12
   gaaaagacgc gctgacaata cgcc 24
<210> 13
   <211> 20
   <212> DNA
   <213> Artificial
<400> 13
   gccatcagca ggcttagccg 20
<210> 14
   <211> 23
   <212> DNA
   <213> Artificial
<400> 14
   gggtattgtg gcatgtttaa ccg 23
<210> 15
   <211> 101
   <212> DNA
   <213> Artificial
<400> 15
   atgcgtcttc ttcataccat gctgcgcgtt ggcgatttgc aacgctccat cgatttttat 60
   accaaagtgc tgggcatgaa gtgtaggctg gagctgcttc g 101
<210> 16
   <211> 100
   <212> DNA
   <213> Artificial
<400> 16
   ttagttgccc agaccgcgac cggcgtcttt ctcttcgatt aactcaattt tgtaaccgtc 60
   cggatcttcc acaaacgcga catatgaata tcctccttag 100
<210> 17
   <211> 31
   <212> DNA
   <213> Artificial
<400> 17
   gaagtggtcg atgccgggat tgaagaatgg g 31
<210> 18
   <211> 31
   <212> DNA
   <213> Artificial
<400> 18
   gggttacgtt tcagtgaggc gcgttctgcg g 31
<210> 19
   <211> 102
   <212> DNA
   <213> Artificial
<400> 19
   atgtcagtac ccgttcaaca tcctatgtat atcgatggac agtttgttac ctggcgtgga 60
   gacgcatgga ttgatgtggt agtgtaggct ggagctgctt cg 102
<210> 20
   <211> 101
   <212> DNA
   <213> Artificial
<400> 20
   ttaagactgt aaataaacca cctgggtctg cagatattca tgcaagccat gtttaccatc 60
   tgcgccgcca ataccggatt tcatatgaat atcctcctta g 101
<210> 21
   <211> 31
   <212> DNA
   <213> Artificial
<400> 21
   tgcagcggcg cacgatggcg acgttccgcc g 31
<210> 22
   <211> 31
   <212> DNA
   <213> Artificial
<400> 22
   cacgatgacg accattcatg cctatactgg c 31
<210> 23
   <211> 101
   <212> DNA
   <213> Artificial
<400> 23
   tcagaacagc cccaacggtt tatccgagta gctcaccagc aggcacttgg tttgctggta 60
   atgctccagc atcatcttgt gtgtaggctg gagctgcttc g 101
<210> 24
   <211> 100
   <212> DNA
   <213> Artificial
<400> 24
   atgaccaata atcccccttc agcacagatt aagcccggcg agtatggttt ccccctcaag 60
   ttaaaagccc gctatgacaa catatgaata tcctccttag 100
<210> 25
   <211> 40
   <212> DNA
   <213> Artificial
<400> 25
   catatttccc tcaaagaata taaaaaagaa caattaacgc 40
<210> 26
   <211> 31
   <212> DNA
   <213> Artificial
<400> 26
   tatgttcatg cgatggcgca ccagctgggc g 31
<210> 27
   <211> 29
   <212> DNA
   <213> Artificial
<400> 27
   cgcgtgatcg acggtgctga tggtgcccg 29
<210> 28
   <211> 19
   <212> DNA
   <213> Artificial
<400> 28
   aagttcagga gagccgccc 19
<210> 29
   <211> 101
   <212> DNA
   <213> Artificial
<400> 29
   gttattccca ctcttgcagg aaacgctgac cgtactggtc ggctaccagc agagcggcgt 60
   aaacctgatc tggcgtcgcg gtgtaggctg gagctgcttc g 101
<210> 30
   <211> 100
   <212> DNA
   <213> Artificial
<400> 30
   atggaccgca ttattcaatc accgggtaaa tacatccagg gcgctgatgt gattaatcgt 60
   ctgggcgaat acctgaagcc catatgaata tcctccttag 100
<210> 31
   <211> 35
   <212> DNA
   <213> Artificial
<400> 31
   gcctggattt gtaccacggt tggtggaacg gcggg 35
<210> 32
   <211> 24
   <212> DNA
   <213> Artificial
<400> 32
   cacgcatatt ccccattgcc gggg 24
<210> 33
   <211> 101
   <212> DNA
   <213> Artificial
<400> 33
   ttaaaaagtg atacaggttg cgccctgttc ggcaccggac agtttttcac gcaaggcgct 60
   gaataattca cgtcctgttc gtgtaggctg gagctgcttc g 101
<210> 34
   <211> 100
   <212> DNA
   <213> Artificial
<400> 34
   atgaatccac aattgttacg cgtaacaaat cgaatcattg aacgttcgcg cgagactcgc 60
   tctgcttatc tcgcccggat catatgaata tcctccttag 100
<210> 35
   <211> 24
   <212> DNA
   <213> Artificial
<400> 35
   ccccggaatc agaggaatag tccc 24
<210> 36
   <211> 29
   <212> DNA
   <213> Artificial
<400> 36
   gggtagactc cattactgag gcgtgggcg 29

## Description

La présente invention concerne un nouveau procédé de préparation d'un microorganisme évolué pour la production de 1,2-propanediol, le microorganisme évolué ainsi obtenu et son utilisation pour la préparation de 1,2-propanediol.

Le 1,2-propanediol ou propylène glycol, dialcool en C3, est un produit chimique utilisé dans de nombreux domaines. C'est un constituant des résines de polyesters insaturés ; des détergents liquides ; des agents de refroidissement, anti-gel et de fluide de dégivrage des avions. L'utilisation du propylène glycol est en émergence depuis les années 1993-1994 en remplacement des dérivés éthyléniques qui sont reconnus comme plus toxiques que les dérivés propyléniques.

Le 1,2-propanediol est actuellement produit par voie chimique par un procédé d'hydratation d'oxyde de propylène utilisant de large quantité d'eau. La production d'oxyde de propylène peut être effectuée selon deux procédés l'un utilisant de l'épichlorydrine, l'autre de l'hydroperoxide. Ces deux voies utilisent des produits fortement toxiques. De plus, la voie de l'hydroperoxide génère des coproduits tels que le ter-butanol ou le 1 phenyl éthanol qu'il est nécessaire de valoriser pour rentabiliser la production d'oxyde de propylène. La voie chimique produit généralement du 1,2-propanediol racémique alors qu'il existe deux formes de stéréoisoméres le (R) 1.2 propaendiol et le (S) 1,2-propanediol dont l'intérêt n'est pas négligeable pour d'autres applications.

Ces inconvénients liés à la production chimique du 1,2-propanediol font de la voie de production biologique une alternative prometteuse. Plusieurs microorganismes sont capables de produire naturellement du ( S ) ou ( R ) 1,2-propanediol à partir de sucre, tels que le glucose ou le xylose qui sont métabolisés par la voie de la glycolyse, ou encore, à partir de déoxyhexoses qui conduisent alors à la formation de ( S ) 1,2-propanediol (Cameron D. C. et co/l. (1998) Biotechnol. Prog.). Parmi les microorganismes les plus performants sont à citer *Clostridium sphenoides* (*Tran Din K et co*/*l. 1986*) et *Thermoanaerobium thermosaccharolyticum* (*Altaras N. E and Cameron D. C. 2001*). Ce dernier est capable de fermenter plusieurs types de sucres en (R) 1,2-propanediol avec un rendement qui varie de 0.13 à 0.28 g de 1,2-propanediol produit/ g de glucose consommé. Chez ces deux microroganismes, les enzymes responsables de la synthèse de 1,2-propanediol n'ont pas été identifiées, et l'amélioration de leurs performances est limitée par le manque d'outils génétiques disponibles. Par ailleurs, *E. coli* possède tous les gènes nécessaires à la production de 1,2-propanediol même si *E. coli* ne produit pas naturellement du 1,2-propanediol. En effet, le 1,2-propanediol doit être produit à partir de méthyl glyoxal, un composé fortement toxique pour la cellule même à faible concentration. Aussi, des procédés utilisant des souches d'*E. coli* génétiquement modifiées afin qu'elles produisent du 1,2-propanediol ont été décrits notamment dans US 6 303 352, US 6 087 140 et WO 98/37204. Ces procédés utilisent notamment la surexpression d'une ou plusieurs enzymes impliquées dans la voie métabolique de production du 1,2-propanediol par clonage de leurs gènes dans des plasmides et donc imposent une pression de sélection à l'aide d'antibiotiques. Pour améliorer les performances des souches certains gènes endogènes sont également délétés (voir par exemple Altaras N. E. and Cameron D. C. (2000) Biotechnol. Prog. 16, 940-946*:* Altaras N. E. and Cameron D. (1999) Appl. Env. Microb., 65, 1180-1185).

Un procédé utilisant un microorganisme évolué coproduisant du 1,2-propanediol et de l'acétone, deux coproduits valorisables, n'a jusqu'alors jamais été décrit.

La présente invention concerne donc un procédé de préparation d'une souche de microorganismes évolués pour la production de 1,2-propanediol par métabolisme d'une source de carbone simple, à partir d'une souche initiale comprenant une délétion du gène *tpiA* et une délétion d'au moins un gène codant pour une enzyme impliquée dans la conversion du méthyl glyoxal (propanal) en lactate ledit procédé comprenant les étapes suivantes de :
- cultiver ladite souche initiale dans un milieu de culture approprié, comprenant une source de carbone simple, en appliquant des taux de dilution croissants de telle sorte de ne conserver dans le milieu de culture que les microorganismes ayant un taux de croissance égal ou supérieur au taux de dilution imposé , afin de faire évoluer dans ladite souche initiale un ou plusieurs gènes impliqués dans la voie de biosynthèse du DHAP en méthylglyoxal puis en 1,2-propanediol vers des gènes évolués ayant une activité « 1,2-propanediol synthase » améliorée,
- sélectionner et isoler la ou les souches de microorganismes évolués ayant une activité « 1,2-propanediol synthase » améliorée.

Le gène *tpiA* code pour la triose phosphate isomérase qui catalyse la conversion du DHAP en glycéraldéhyde 3 phosphate. La délétion de ce gène a pour objet d'assurer la synthèse d'une quantité suffisante de méthyl glyoxal. Théoriquement, la délétion du gène *tpiA* doit permettre d'assurer que 50% du carbone du glucose métabolisé par les cellules soit affecté à la préparation du méthyl glyoxal à partir du dihydroxy acétonephosphate.

La délétion d'au moins un gène impliqué dans la conversion du méthyl glyoxal (propanal) en lactate a pour objet d'inhiber la conversion du méthyl glyoxal en lactate, de manière que l'essentiel du méthyl glyoxal présent et produit par la souche initiale, comme par la souche évoluée obtenue, soit employé par la machinerie cellulaire desdites souches pour la préparation de 1,2-propanediol.

Les gènes impliqués dans la conversion du méthyl glyoxal en lactate sont choisis parmi le gène *gloA* codant pour la glyoxylase I (catalysant la synthèse de lactoyl glutathione à partir de méthylglyoxal) et les gènes *aldA* et *aldB* codant pour une lactaldéhyde déshydrogénase (catalysant la synthèse de (S) lactate à partir de (S) lactaldéhyde). De préférence, la souche initiale comprend la délétion des trois gènes *gloA, aldA* et *aldB*.

De manière avantageuse, on effectue une modification supplémentaire de la souche initiale qui consiste à supprimer les voies naturelles de fermentation du glucose qui sont consommatrices d'équivalents réducteurs sous forme de NADH afin d'éliminer ces voies métaboliques qui sont en compétition avec la production de 1,2-propanediol.

On citera en particulier la délétion du gène *ldhA* codant pour la lactate déshydrogénase catalysant la synthèse de lactate à partir de pyruvate, et celle du gène *adhE* codant pour l'alcool-aldéhyde déshydrogénase catalysant la synthèse d'éthanol à partir d'acétyl-CoA.

De même, on peut obliger le microorganisme à utiliser le complexe pyruvate déshydrogénase pour produire, en anaérobiose, de l'acétyl-CoA et du NADH à partir du pyruvate. Ceci peut être obtenu en délétant les gènes *pflA* et, *pflB* codant pour la pyruvate formate lyase.

Selon un mode particulier de réalisation de l'invention, la souche initiale comprend donc également une délétion d'au moins un gène choisi parmi *ldhA, pflA, pflB* et *adhE*, de préférence la délétion des quatre gènes *ldhA, pflA, pflB* et *adhE*.

De manière encore plus avantageuse, la souche initiale selon l'invention comprendra également au moins un gène codant pour une enzyme favorisant en anaérobiose, le métabolisme du pyruvate en acétate.

De préférence, l'enzyme favorise, en anaérobiose, le métabolisme du pyruvate vers la production d'acétyl-CoA et de NADH. Plus préférentiellement cette enzyme est un complexe pyruvate déshydrogénase.

De manière avantageuse, ledit gène codant pour une enzyme favorisant, en anaérobiose, le métabolisme du pyruvate en acétate est peu sensible à l'inhibition par le NADH.

Ce gène peut être un gène endogène, codant pour une protéine endogène, ou encore un gène exogène ou hétérologue, codant pour une enzyme endogène ou exogène.

Dans le cas d'un gène endogène codant pour une protéine endogène sensible à l'inhibition par le NADH, le procédé d'évolution selon l'invention permettra de sélectionner les souches à activité « 1,2-propanediol synthase » améliorée dont ledit gène codant pour une enzyme favorisant, en anaérobiose, le métabolisme du pyruvate en acétate code pour une enzyme évoluée rendue peu sensible à l'inhibition par le NADH.

Selon un autre mode de réalisation de l'invention, on peut introduire dans la souche initiale un gène hétérologue qui code pour une enzyme peu sensible à l'inhibition par le NADH, ou codant pour une enzyme sensible, mais rendue peu sensible par la mise en oeuvre du procédé d'évolution selon l'invention.

En outre, il est avantageux de déléter également le gène *edd* codant pour la 6-phospho-gluconate deshydratase, première enzyme impliquée dans la voie d'Entner Doudoroff, pour éviter la métabolisation directe du glucose en glycéraldéhyde-3-phosphate et pyruvate et ainsi forcer la conversion du glucose en 1,2 propanediol et acétate

De manière avantageuse, on introduit dans la souche évoluée préalablement isolée, obtenue par le procédé d'évolution selon l'invention, un ou plusieurs gènes hétérologues codant pour une ou plusieurs enzymes impliquées dans la conversion de l'acétyl-CoA et de l'acétate en acétone, pour obtenir une souche évoluée modifiée.

Cette nouvelle modification permet de produire avec le 1,2-propanediol de l'acétone, coproduit valorisable. Cette modification a en outre l'avantage d'améliorer les performances de production en 1,2-propanediol. En effet, l'acétate est un composé inhibiteur de la croissance bactérienne à faible concentration (15 g/l) et bloque rapidement l'évolution des performances de la souche cultivée en chemostat en conditions anaérobies.

L'introduction dans la souche évoluée des gènes codant pour les enzymes catalysant la transformation de l'acétate en acétone entraîne une diminution de la concentration résiduelle en acétate lors de la culture en chemostat. De l'acétone est produit, composé largement moins inhibiteur de la croissance que l'acétate, la croissance de la souche et la production de 1,2-propanediol sont favorisées.

De manière avantageuse, le ou les gènes hétérologues codant pour une ou plusieurs enzymes impliquées dans la conversion de l'acétyl-CoA et de l'acétate proviennent de *C. acetobutylicum*. Les gènes codant pour une ou plusieurs enzymes impliquées dans la conversion de l'acétyl-CoA et de l'acétate en acétone peuvent être exprimés chromosomiquement ou extrachromosomiquement. Chromosomiquement, une ou plusieurs copies peuvent être introduites dans le génome à l'aide des méthodes de recombinaison connues de l'homme de l'art. Extrachromosomiquement, les gènes peuvent être portés par différents types de plasmides qui diffèrent par leur origine de réplication, leur nombre de copies et leur stabilité dans la cellule. Ils peuvent être présents de 1 à 5 copies, comme à 20 ou jusqu'à plus de 500 copies correspondant aux plasmides à bas nombre de copies avec une origine de réplication de type strict (pSC101, RK2), aux plasmides à bas nombre de copie (pACYC, pRSF1010) ou à grand nombre de copies (pSK bluescript II). Les gènes peuvent être exprimés en utilisant des promoteurs de différentes forces, inductibles ou non inductibles. On peut citer par exemple les promoteurs Ptrc, Ptac, Plac, ou d'autres promoteurs connus de l'homme de l'art. L'expression des gènes cibles peut être augmentée ou diminuée par des éléments stabilisant ou destabilisant l'ARN messager (Carrier and Keasling (1998) Biotechnol. Prog., 15, 58-64) ou les protéines (par exemple GSTtags, Amersham Biosciences).

Selon un mode préférentiel de réalisation de l'invention, on cultive la souche évoluée modifiée obtenue précédemment sous pression de sélection dans un milieu de culture approprié comprenant une source de carbone simple afin de faire évoluer dans ladite souche évoluée modifiée un ou plusieurs gènes impliqués dans la conversion de l'acétyl-CoA et de l'acétate en acétone vers une activité « acétone synthase » améliorée, puis on sélectionne et on isole les souches de microorganismes évolués de deuxième génération ayant une activité « 1,2-propanediol synthase » améliorée et une activité « acétone synthase » améliorée.

La présente invention concerne aussi une souche initiale selon l'invention telle que définie ci-dessus, ci-après et dans les exemples qui comprend également une délétion des gènes *aldA* et/ou *aldB* et/ou dans laquelle les voies naturelles de fermentation du glucose qui sont consommatrices de NADH sont supprimées et/ou qui comprend également la délétion des gènes *adhE* et *ldha* et/ou dans laquelle le gène *lpd* codant pour la lipoamide dehydrogenase du complexe pyruvate déshydrogénase porte une mutation ponctuelle entraînant une modification de l'alamine 55 en valine et/ou comprend également une délétion des gènes *pflA* et *pflB* et/ou du gène *edd*.

Elle concerne également une souche évoluée ayant une activité « 1,2-propanediol synthase » améliorée dans laquelle le gène *lpd* porte une mutation ponctuelle entrainant une modification de l'alanine 55 en valine cette définition englobant les souches évoluées de deuxième génération qui ont au surplus une activité « acétone synthase » améliorée.

L'invention concerne enfin un procédé de préparation de 1,2-propanediol dans lequel on cultive une souche évoluée dans laquelle le gène *lpd* porte une mutation ponctuelle entrainant une modification de l'alanine 55 en valine dans un milieu de culture approprié comprenant une source simple de carbone, puis on récupère le 1,2-propanediol produit et le cas échéant de l'acétone, qui sont éventuellement purifiés.

Les souches de microorganismes modifiés, initiaux et évolués, selon l'invention peuvent être procaryotiques ou eucaryotiques, susceptibles d'être transformés et cultivés pour permettre la production de 1,2-propanediol et le cas échéant d'acétone.

L'homme du métier sera à même de sélectionner lesdits microorganismes au regard de ses connaissances générales dans le domaine de la biologie cellulaire et moléculaire, et, le cas échéant, d'identifier les gènes de ces microorganismes correspondant aux gènes de *E. coli* mentionnés précédemment.

Par souche de microorganismes, on entend selon l'invention un ensemble de microorganismes d'une même espèce comprenant au moins un microorganisme de ladite espèce. Ainsi, les caractéristiques décrites pour la souche s'appliquent à chacun des microorganismes de ladite souche. De même, les caractéristiques décrites pour l'un des microorganismes de la souche s'appliqueront à l'ensemble desdits microorganismes la composant.

Les microorganismes modifiés selon l'invention sont choisis parmi les bactéries, les levures et les champignons, et notamment ceux des espèces suivantes : *Aspergillus sp., Baci*/*lus sp., Brevibacterium sp., Clostridium sp., Corynebacterium sp., Escherichia sp., Gluconobacter sp., Pseudomonas sp., Rhodococcus sp., Saccharomyces sp., Streptomyces sp., Xanthomonas sp., Candida sp.*

Dans un mode de réalisation préféré, la souche bactérienne est une souche d'*Escherichia*, en particulier d'*E. coli*. Dans un autre mode de réalisation, la souche bactérienne est une souche de *Corynebacterium*, en particulier *C. glutamicum*.

Dans un autre mode de réalisation, la souche de levure est une souche de *Saccharomyces*, en particulier *S. cerevisiae*

L'invention est décrite ci-dessus, ci-après et dans les exemples par rapport à *E. coli*. Ainsi, les gènes susceptibles d'être délétés ou surexprimés pour les souches évoluées selon l'invention sont définis principalement par l'emploi de la dénomination du gène de *E. coli*. Cependant, cet emploi a une signification plus générale selon l'invention et englobe les gènes correspondants d'autres microorganismes. En effet en utilisant les références GenBank des gènes d'*E. coli*, l'homme du métier est capable de déterminer les gènes équivalents dans d'autres souches bactériennes qu'*E. coli*.

Les moyens d'identification des séquences homologues et de leurs pourcentages d'homologie sont bien connus de l'homme du métier, comprenant notamment les programmes BLAST qui peuvent être utilisés à partir du site http://www.ncbi.nlm.nih.gov/BLAST/ avec les paramètres indiqués par défaut sur ce site. Les séquences obtenues peuvent alors être exploitées (e.g. alignées) en utilisant par exemple les programmes CLUSTALW (http://www.ebi.ac.uk/clustalw/) ou MULTALIN (http://prodes.toulouse.inra.fr/multalin/cgi-bin/multalin.pl), avec les paramètres indiqués par défaut sur ces sites.

En utilisant les références données sur GenBank pour les gènes qui sont connus, l'homme du métier est capable de déterminer les gènes équivalents dans d'autres organismes, souches bactériennes, levures, champignons, mammifères, plantes, etc. Ce travail de routine est avantageusement effectué en utilisant les séquences consensus pouvant être déterminées en réalisant des alignements de séquences avec des gènes issus d'autres microorganismes, et en dessinant des sondes dégénérées permettant de cloner le gène correspondant dans un autre organisme. Ces techniques de routine de biologie moléculaire sont bien connues dans l'art et sont décrites par exemple dans Sambrook et al. (1989 Molecular cloning: a laboratory manual. 2nd Ed. Cold Spring Harbor Lab., Cold Spring Harbor, New York.).

Par « délétion », on entend selon l'invention une suppression de l'activité du gène « délété ». Cette suppression peut être une inactivation du produit d'expression du gène concerné par un moyen approprié, ou bien l'inhibition de l'expression du gène concerné, ou encore la délétion d'au moins une partie du gène concerné de manière soit que son expression n'ait pas lieu (par exemple délétion d'une partie ou de l'ensemble de la région promotrice nécessaire à son expression) soit que le produit d'expression ait perdu sa fonction (par exemple délétion dans la partie codante du gène concerné). De manière préférentielle, la délétion d'un gène comprend la suppression de l'essentiel dudit gène, et le cas échéant son remplacement par un gène marqueur de sélection permettant de faciliter l'identification, l'isolement et la purification des souches évoluées selon l'invention.

L'inactivation d'un gène se fait préférentiellement par recombinaison homologue. (Datsenko, K.A.; Wanner, B.L. (2000) One-step inactivation of chromosomal genes in Escherichia coli K-12 using PCR products. Proc. Natl. Acad. Sci. USA 97 : 6640-6645). Le principe d'un protocole en est rappelé brièvement : un fragment d'ADN linéaire est introduit dans la cellule, lequel fragment est obtenu in vitro, comprenant les deux régions flanquant le gène, et au moins un gène de sélection entre ces deux régions (généralement un gène de résistance à un antibiotique), ledit fragment linéaire présentant donc un gène inactivé. Les cellules ayant subi un événement de recombinaison et ayant intégré le fragment introduit sont sélectionnées par étalement sur milieu sélectif. On sélectionne ensuite les cellules ayant subi un événement de double recombinaison, dans lesquelles le gène natif a été remplacé par le gène inactivé. Ce protocole peut être amélioré en utilisant des systèmes de sélections positive et négative, afin d'accélérer la détection des événements de double recombinaisons.

La technique préférentiellement utilisée pour l'introduction de ces gènes dans la souche est l'électroporation, technique bien connue de l'homme du métier. Le protocole en est rappelé brièvement : les gènes hétérologues d'intérêt sont clonés dans un vecteur d'expression entre un promoteur et un terminateur. Ce vecteur possède en outre un gène de résistance à un antibiotique afin de sélectionner les cellules le contenant et une origine de réplication fonctionnelle chez la souche hôte afin qu'il puisse se maintenir. Le protocole nécessite la préparation de cellules hôtes électrocompétentes qui sont ensuite transformées par électroporation par le vecteur.

Selon l'invention, les gènes introduits par électroporation sont préférentiellement les gènes *adc, ctfA* et *B, thl* codant respectivement pour l'acétoacétate carboxylase, la coenzyme A transférase et la thiolase de la voie naturelle de production d'acétone de *Clostridium acetobutylicum*, microorganisme reconnu comme extrêmement performant pour la production d'acétone par voie biologique.

Le procédé d'évolution selon l'invention est un procédé de préparation de microorganismes évolués permettant une modification des voies métaboliques, qui comprend de préférence les étapes suivantes :
a) Modification d'un microorganisme pour obtenir un microorganisme initial de manière à inhiber la production ou la consommation d'un métabolite autrement consommé ou produit lorsque les cellules du microorganisme initial est cultivé sur un milieu défini,
b) Culture des microorganismes initiaux modifiés précédemment obtenus sur ledit milieu défini pour le faire évoluer, le milieu défini pouvant également comprendre un co-substrat nécessaire à cette évolution,
c) Sélection des cellules de microorganismes modifiés capables de se développer sur le milieu défini, éventuellement avec un co-substrat.

Un tel procédé d'évolution est décrit notamment dans la demande de brevet WO 04/076659.

En l'espèce, la voie métabolique évoluée est la voie de biosynthèse du 1,2-propanediol, et le cas échéant la voie de biosynthèse de l'acétone.

Par « milieu défini », on entend selon l'invention un milieu de composition moléculaire connue, adapté à la croissance du microorganisme. Le milieu défini est substantiellement exempt de métabolite dont on supprime la production ou la consommation en réalisant la modification.

Par « co-substrat », on entend selon l'invention une molécule carbonée ou non, différent du substrat, qui est impliqué dans une réaction et donnant un ou plusieurs atomes au substrat afin de former le produit. Le co-substrat n'a pas de propriété mutagène reconnue.

Par « sélection », on entend selon l'invention un procédé de culture, éventuellement en continu, conduit en appliquant des taux de dilution croissants de telle sorte de ne conserver dans le milieu de culture que les microorganismes ayant un taux de croissance égal ou supérieur au taux de dilution imposé. Ce faisant, on conserve les microorganismes ayant évolués de telle sorte que la modification réalisée n'affecte plus la croissance.

Par « gène évolué », on entend selon l'invention une succession d'acide nucléique délimité par un codon start et un codon stop en phase et ayant, à l'issue de la sélection, au moins un acide nucléique différent par rapport à la séquence initiale.

Par « protéine évoluée », on entend selon l'invention une succession d'acides aminés (séquence protéique) ayant, à l'issue de la sélection, au moins un acide aminé différent par rapport à la séquence protéique initiale.

Les gènes et protéines peuvent être identifiées par leurs séquences primaires, mais également par homologies de séquences ou alignements qui définissent des groupes de protéines.

Les PFAM (Protein families database of alignments and Hidden Markov Models ; http://www.sanger.ac.uk/Software/Pfam/) représentent une large collection d'alignements de séquences protéiques. Chaque PFAM permet de visualiser des alignements multiples, de voir des domaines protéiques, d'évaluer la répartition entre les organismes, d'avoir accès à d'autres bases de données, de visualiser des structures connues de protéines.

Les COGs (Clusters of Orthologous Groups of proteins ; http://www.ncbi.nlm.nih.gov/COG/) sont obtenus en comparant les séquences protéiques issus de 43 génomes complètement séquencés représentant 30 lignées phylogénétiques majeurs. Chaque COG est défini à partir d'au moins trois lignées ce qui permet ainsi d'identifier des domaines conservés anciens.

Selon l'invention, les termes « culture » et « fermentation » sont employés indifféremment pour désigner la croissance de la bactérie sur un milieu de culture approprié comprenant une source de carbone simple.

Par source de carbone simple, selon la présente invention, on entend des sources de carbone utilisables par l'homme du métier pour la croissance normale d'un microorganisme, d'une bactérie en particulier qui peuvent être l'arabinose, le fructose, le galactose, le lactose, le maltose, le sucrose et le xylose. Une source de carbone simple tout particulièrement préférée est le glucose.

La définition des conditions de culture des microorganismes selon l'invention (fermentation) est à la portée de l'homme du métier. On fermente notamment les bactéries à une température comprise entre 20°C et 55°C, de préférence entre 25°C et 40°C, plus particulièrement d'environ 30°C pour C. *glutamicum* et *S. cerevisiae* et d'environ 34°C pour *E. coli*.

La fermentation est généralement conduite en fermenteurs avec un milieu minéral de culture de composition connu défini et adapté en fonction des bactéries utilisées, contenant au moins une source de carbone simple et le cas échéant un cofacteur nécessaire à la production du métabolite.

En particulier, le milieu minéral de culture pour *E. coli* pourra ainsi être de composition identique ou similaire à un milieu M9 (Anderson, 1946, Proc. Natl. Acad. Sci. USA 32:120-128), un milieu M63 (Miller, 1992 ; A Short Course in Bacterial Genetics: A Laboratory Manual and Handbook for Escherichia coli and Related Bacteria, Cold Spring Harbor Laboratory Press, Cold Spring Harbor, New York) ou un milieu tel que défini par Schaefer et al. (1999, Anal. Biochem. 270 : 88-96), plus particulièrement le milieu de culture minimum décrit ci-dessous :

| | |
|---|---|
| K₂HPO₄ | 1g/l |
| N.T.A | 0,2g/l |
| solution d'oligoélément* | 10ml/l |
| (NH₄)₂SO₄ | 1g/l |
| NaCl | 0,2g/l |
| NaHCO₃ | 0,2g/l |
| MgSO₄ | 0,2g/l |
| glucose | 20 à 100g/l |
| nitrate de sodium | 0,424g/l |
| thiamine | 10m/l |
| FeSO4 | 50mg/l |
| Extrait de levure | 4g/l |
| spectinomycine | 100mg/l |

| | |
|---|---|
| Le pH du milieu est ajusté à 7,4 avec de la soude. **solution d'oligoélément :* Acide citrique à 4g/L, Mn SO4 à 3g/L, Na Cl à 1g/L, Co Cl2 à 0,1g/L, Zn SO4 à 0,10g/L, Cu SO4 à 10mg/L, H3 BO3 à 10mg/L, Na Mo 04 à 10mg/L. | |

De manière analogue, le milieu minéral de culture pour *C. glutamicum* pourra ainsi être de composition identique ou similaire au milieu BMCG (Liebl et al., 1989, Appl. Microbiol. Biotechnol. 32: 205-210) à un milieu tel que défini par Riedel et al. (2001, J. Mol. Microbiol. Biotechnol. 3: 573-583).

La fermentation est préférentiellement réalisée en anaérobiose et en chemostat, c'est à dire, alimentée en continu, à un taux de dilution fixe, avec ledit milieu de culture minimum contenant une concentration en source de carbone fixe et étant dégazé à l'azote.

La concentration en source de carbone du milieu d'alimentation de la fermentation n'est augmentée que lorsque un régime permanent limité par la concentration en source de carbone résiduelle est atteint et stable pendant plusieurs jours.

Le mode de culture en chemostat est le mode de culture préférentiel car c'est celui qui favorise l'amélioration des performances de croissance et de production en 1,2-propanediol de la souche modifiée et conduit à isoler les microorganismes évolués.

Par activité « 1,2-propanediol synthase » améliorée, on entend selon l'invention l'ensemble des activités enzymatiques améliorées impliquées dans la voie de conversion du DHAP en 1,2-propanediol. L'activité enzymatique améliorée dans le microorganisme évolué consiste en une augmentation de la quantité de 1,2-propanediol par le microorganisme évolué par rapport aux quantités produites par le microorganisme initial correspondant, dans des conditions de culture identiques.

Par activité activité « acétone synthase » améliorée, on entend selon l'invention, l'ensemble des activités enzymatiques améliorées impliquées dans la voie de conversion de l'acétate et de l'acétyl-coA en acétone. L'activité enzymatique évoluée dans le microorganisme évolué de deuxième génération consiste en une augmentation de la quantité d'acétone produite par le microorganisme évolué de deuxième génération par rapport au microorganisme évolué transformé correspondant, dans des conditions de culture identiques.

L'invention concerne aussi l'isolement et la caractérisation des gènes évolués dans les souches évoluées obtenues par le procédé selon l'invention, et des protéines évoluées codées par lesdits gènes évolués. Ces gènes évolués peuvent être ensuite introduits dans un organisme hôte sous le contrôle d'éléments de régulation appropriés pour son expression dans ledit organisme afin de permettre la production de la protéine évoluée correspondante.

L'amélioration de performances des microorganismes modifiés, en particulier de la souche *E. coli* MG16555 Δ *tpiA,* Δ*pflAB,* Δ*adhE, ldhA:: kana,* Δ*g*/*oA,* Δ*aldA,* Δ*aldB* au cours de la culture en chemostat suggère que ces conditions de culture permettent de sélectionner un complexe pyruvate déshydrogénase endogène fonctionnel en conditions d'anaérobioses, conditions de fortes productions de NADH. Il est en effet connu que le complexe pyruvate déshydrogénase catalysant la transformation du pyruvate en acétyl-CoA en libérant du NADH n'est fonctionnel qu'en aérobie, alors que lorsqu'on est en condition d'anaérobiose c'est la pyruvate formate lyase qui est fonctionnelle et catalyse la transformation du pyruvate en acétyl-CoA et formate (Snoep J. L., De Graef M. R., Westphal A. H., De Kok A. Teixeira de Mattos M. J. and Neijssel O. M. (1993). Or une des modifications effectuées, sur la souche modifiée d'*E. coli* construite pour la production de 1,2-propanediol, pour produire du NADH par décarboxylation du pyruvate, est la déplétion des gènes *pflA* et *pflB* codant pour l'activité pyruvate formate lyase. La seule possibilité pour la cellule modifiée est de métaboliser le pyruvate en acétyl-CoA par le complexe pyruvate déshydrogénase en produisant un NADH. Le complexe pyruvate déshydrogènase de la souche modifiée évoluée a été caractérisé et est moins sensible au NADH que le complexe pyruvate déshydrogènase de la souche sauvage.

La présente invention conduit à la sélection d'un complexe pyruvate déshydrogénase fonctionnel en anaérobiose qui permet de produire deux NADH par oxydation du glycéraldéhyde-3-Phosphate en acétate, NADH qui ne peuvent être réoxydés que par la voie de réduction du dihydroxyacétone-phosphate en 1,2-propanediol. La sélection d'un complexe enzymatique peu sensible au NADH favorise la vitesse de production du 1,2-propanediol.

La présente invention conduit avantageusement à la sélection de mutations du gène *lpd* (dont la séquence sauvage est connue hppt://genolist.pasteur.fr/Colibri) codant pour la lipoamide déshydrogénase du complexe pyruvate déshydrogénase. En particulier, la présence d'une mutation ponctuelle entraînant le remplacement de l'alanine 55 par une valine a été identifié. Cette enzyme est connue pour être responsable de l'inhibition du complexe pyruvate déshydrogénasse par le NADH. Cette enzyme modifiée est également partie de la présente invention.

La présente invention permet l'amélioration des performances des microorganismes modifiés, en particulier de la souche *E. coli* MG1655 Δ *tpiA,* Δ*pflAB,* Δ*adhE, ldhA:: kana,* Δ*gloA,* Δ*a*/*dA,* Δ*aldB* également, par évolution, au cours de la culture anaérobie en chemostat, des enzymes endogènes impliquées dans la voie de conversion du DHAP en 1,2-propanediol. L'évolution de ces enzymes a pour conséquence une augmentation du taux de croissance et de la concentration finale en 1,2-propanediol.

De manière préférentielle selon l'invention la souche évoluée ne comprend pas l'évolution du gène *gldA*. Selon un mode particulier la souche d'évolution comprend une délétion du gène *gldA*.

### Description des figures

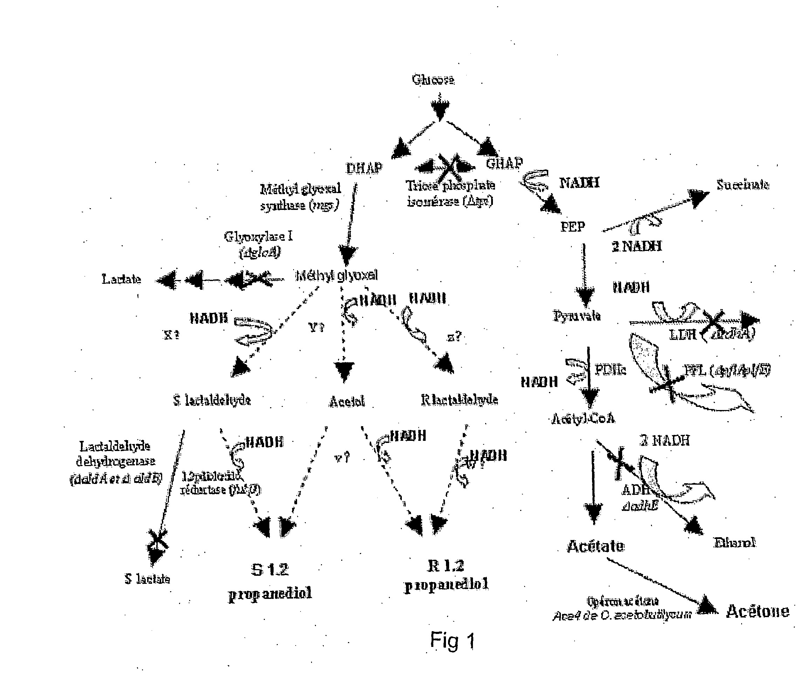

Figure 1 : schéma du métabolisme de la souche modifiée *E. coli* pour la production de 1,2-propanediol et d'acétone selon l'invention
   Légende :
   LDH : lactate déshydrogénase
   ADH : aldéhyde-alcool déshydrogénase
   PFL : pyruvate formate lyase
   PDHc : complexe pyruvate déshydrogénase
Figure 2 : Evolution de la souche *E. coli* MG1655 Δ *tpiA,* Δ*pflAB,* Δ*adhE, ldhA:: kana,* Δ*gloA,* Δ*a*/*dA,* Δ*aldB* en culture chemostat sur glucose : concentration de glucose (figure 2A et autres produits (figure 2B).
Figure 3 : comparaison de l'activité enzymatique du complexe pyruvate déshydrogénase de la souche sauvage et de la souche évoluée selon l'invention en fonction de concentrations croissantes en NADH.

Les exemples de réalisation ci-dessous permettrent d'illustrer l'invention, sans chercher à en limiter la portée.

Exemple 1 : construction d'une souche modifée d'*E. coli* MG1655Δ*tpiA*, Δ*pflAB,* Δ*adhE, ldhA ::kana,* Δ*gloA,* Δ*aldA,* Δ*aldB* capable de produire uniquement du 1,2-propanediol et de l'acétate par fermentation du glucose :

### a) construction d'une souche modifiée E. coli MG1655Δ tpiA :: cm :

L'inactivation du gène *tpiA* est réalisée en insérant une cassette de résistance à l'antibiotique chloramphénicol tout en délétant la majeure partie du gène concerné. La technique utilisée est décrite par Datsenko, K.A. ; Wanner, B.L. (2000) One-step inactivation of chromosomal genes in Escherichia coli K-12 using PCR products. Proc. Natl. Acad Sci. USA 97 : 6640-6645.

Deux oligonucléotides sont utilisés pour réaliser le remplacement du gène *tpiA*: avec :
une région (caractères minuscules) homologue à la séquence (4109007-4109087) du gène *tpiA* (séquence 4108320 à 4109087), séquence de référence sur le site http://genolist.pasteur.fr/Colibri/)
une région (caractères majuscules) pour l'amplification de la cassette de résistance au chloramphénicol du plasmide pKD3 (Datsenko, K.A. ; Wanner, B.L. (2000) One-step inactivation of chromosomal genes in Escherichia coli K-12 using PCR products. Proc. Natl. Acad Sci. USA 97 : 6640-6645) avec :
une région (caractères minuscules) homologue à la séquence (4108320-4108400) du gène *tpiA*
une région (caractères majuscules) pour l'amplification de la cassette de résistance au chloramphénicol portée par le plasmide pKD3

Les oligonucléotides DtpiAr et DtpiAf sont utilisés pour amplifier la cassette de résistance au chloramphénicol à partir du plasmide pKD3. Le produit PCR obtenu est alors introduit par électroporation dans la souche MG1655 (pKD46) dans laquelle le système λ Red (γ, β, exo) exprimé favorise grandement la recombinaison homologue. Les transformants résistants à l'antibiotique sont alors sélectionnés et l'insertion de la cassette de résistance est vérifiée par une analyse PCR avec les oligonucléotides cdh et YIIQ.
cdh (SEQ ID NO 3) :
ggtgatgatagttatcgccg (homologue à la séquence de 41 07536 à 4107555)
YIIQ (SEQ ID NO 4) :
cgtgccatcgacagcagtcc (homologue à la séquence de 4109599 à 4109580)

La cassette de résistance au chloramphénicol est ensuite éliminée. Le plasmide pCP20 porteur de la recombinase FLP agissant au niveau des sites FRT de la cassette de résistance au chloramphénicol, est alors introduit dans les souches recombinantes par électroporation (Cheperanov P. P. and Wackernagel W. (1995) Gene disruption in Escherichia coli: TcR and KmR cassettes with option of Flp-catalyzed excision of the antibiotic-resistance determinant, gene, 158, 9-14 ). Après une série de culture à 42°C, la perte de la cassette de résistance à l'antibiotique est vérifiée par une analyse PCR avec les mêmes oligonucléotides que ceux utilisés précédemment.

### b) construction d'une souche modifiée E. coli MG1655 ΔplfAB :: cm :

L'inactivation des gènes *plfA* et *pflB* est réalisée en insérant une cassette de résistance à l'antibiotique chloramphénicol tout en délétant la majeure partie des gènes concernés. La technique utilisée est décrite par Datsenko, K.A. et Wanner, B.L. (2000).

Deux oligonucléotides sont utilisés pour réaliser le remplacement des gènes *pfla* et *pflB* : avec :
une région (caractères minuscules) homologue à la séquence (952235-952315) du gène *plfB* (séquence 950495 à 952777), séquence de référence sur le site http://jenolist.pasteur.fr/Colibri/)
une région (caractères majuscules) pour l'amplification de la cassette de résistance au chloramphénicol du plasmide pKD3 (Datsenko, K.A. ; Wanner, B.L. (2000) One-step inactivation of chromosomal genes in Escherichia coli K-12 using PCR products. Proc. Natl. Acad Sci. USA 97 : 6640-6645)
avec :
une région (caractères minuscules) homologue à la séquence (949470-949550) située en amont du gène *pflA* (séquence de 949563 à 950303)
une région (caractères majuscules) pour l'amplification de la cassette de résistance au chloramphénicol portée par le plasmide pKD3

Les oligonucléotides pflAB1 et pflAB2 sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides pflAB1 et pfIAB2.
pflAB 1 (SEQ ID NO 7) :
agacattaaaaatatacgtgcagctacccg (homologue à la séquence de 948462 à 948491)
pflAB 2 (SEQ ID NO 8) :
gtgaaagctgacaacccttttgatctttta (homologue à la séquence de 953660 à 983689)

### c) construction d'une souche modifiée E. coli MG1655 ΔtpiA, ΔplfAB :

La délétion des gènes *pflA* et *pf*/*b* par remplacement des gènes par une cassette de résistance au chloramphenicol dans la souche MG1655 Δ*tpiA* a été réalisée par la technique de transduction avec le phage P1. Le protocole est constitué de deux étapes, d'une part la préparation du lysat de phage sur la souche MG1655 Δ*plfAB ::cm* et d'autre part, la transduction de la souche MG1655 Δ*tpiA* par ce lysat de phage.

### Préparation du lysat de phage :

- Ensemencement par 100µl d'une culture de nuit de la souche MG1655 (Δ*plfAB*::cm) de 10 ml de LB + Cm 30µg/ml + glucose 0,2% + CaCl₂ 5 mM
- Incubation 30 min à 37°C sous agitation
- addition de 100 µl de lysat de phage P1 préparé sur la souche sauvage MG1655 (environ 1.10⁹ phage/ml)
- Agitation à 37°C pendant 3 heures jusqu'à la lyse totale des cellules
- Ajout de 200 µl de chloroforme et vortex
- Centrifugation 10 min à 4500g pour éliminer les débris cellulaires
- Transfert du surnageant dans un tube stérile et ajout de 200 µl de chloroforme
- Conservation du lysat à 4°C

### Transduction

- Centrifugation 10 min à 1500g de 5 ml d'une culture de nuit de la souche MG1655 (Δ*tpiA*) en milieu LB
- Suspension du culot cellulaire dans 2,5 ml de MgSO₄ 10 mM, CaCl₂ 5 mM
- Tubes témoins : 100 µl cellules 100 µl phages P1 de la souche MG1655 (Δ*pflAB*::cm)
- Tube test : 100 µl de cellules + 100 µl de phages P1 de la souche MG1655 (Δ*pf*/*AB*::cm)
- Incubation 30 min à 30°C sans agitation
- Ajout de 100 µl de citrate de sodium 1 M dans chaque tube puis vortex
- Ajout de 1 ml de LB
- Incubation 1 heure à 37°C sous agitation
- Etalement sur des boîtes LB + Cm 30 µg/ml après centrifugation 3 min à 7000 rpm des tubes.
- Incubation à 37°C jusqu'au lendemain

### Vérification de la souche

Les transformants résistants à l'antibiotique sont alors sélectionnés et l'insertion de la région contenant (*pflAB*::cm) est vérifiée par une analyse PCR avec les oligonucléotides pflAB1 et pflAB2 d'une part, et cdh et YIIQ d'autre part, afin de vérifier également la délétion du gène *tpiA* dans la souche Δp*flAB ::cm*. La souche retenue est dénommée MG1655 Δ(*pflAB*::cm, Δ*tpiA)*.

Comme précédemment, la cassette de résistance au chloramphénicol est ensuite éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment. La souche obtenue est appelée MG16555 Δ tpiA, ΔpflAB.

### d) construction d'une souche modifiée E. coli MG1655 ΔadhE :: cm:

Comme précédemment l'inactivation du gène *adhE* est réalisée en insérant une cassette de résistance à l'antibiotique chloramphénicol tout en délétant la majeure partie du gène concerné par la technique décrite par Datsenko, K.A. and Wanner, B.L. (2000).

Deux oligonucléotides sont utilisés pour réaliser la délétion : avec :
une région (caractères minuscules) homologue à la séquence (1297263-1297343) du gène *adhE* (séquence 1294669 à 1297344), séquence de référence sur le site http://genolist.pasteur.fr/Colibri/)
une région (caractères majuscules) pour l'amplification de la cassette de résistance au chloramphénicol du plasmide pKD3 (Datsenko, K.A. and Wanner, B.L. (2000)
avec :
une région (caractères minuscules) homologue à la séquence (1294694-1294774) du gène *adhE*
une région (caractères majuscules) pour l'amplification de la cassette de résistance au chloramphénicol portée par le plasmide pKD3

Les oligonucléotides DadhEr et DadhEf sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides ychGf et adhECr.
ychGf (SEQ ID N° 11) :
ggctcattgcaccaccatccag (homologue à la séquence de 1294357 à 1294378)
adhECr (SEQ ID N° 12) :
gaaaagacgcgctgacaatacgcc (homologue à la séquence de 1297772 à 1297749)

### e) construction d'une souche MG1655 ΔtpiA, ΔpflAB, ΔadhE:

La délétion du gène *adhE* dans la souche MG1655 Δ*tpiA,* Δ*plfAB* est effectuée comme précédemment à l'aide de la technique de transduction à l'aide de phage P1 (voir protocole au c). Le lysat de phage P1 est effectué sur la souche MG1655 Δ*adhE ::cm*, et la transduction de la souche MG1655 Δ*tpiA*Δ*pflAB* est réalisée à l'aide de ce lysat. Les transductants résistants au chloramphenicol sont contrôlés à l'aide des oligonucléotides ychCf et adhECr pour vérifier la mutation du gène *adhE* et aussi à l'aide des oligonucléotides pflAB1 et pflAB2 d'une part, et cdh et YIIQ d'autre part, afin de vérifier également la délétion des gènes *pflA* et *B,* et *tpiA* dans la souche Δ*adhE ::cm*.

Comme précédemment, la cassette de résistance au chloramphénicol est ensuite éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment. La souche obtenue est appelée MG16555 Δ *tpiA,* Δ*pflAB,* Δ*adhE.*

### f ) construction d'une souche modifiée E. coli MG1655 ΔtpiA, ΔpflAB, ΔadhE, ldha :: kana :

L'inactivation du gène *ldhA* (coordonnées 1439878 à 1440867) dans la souche MG1655 Δ*tpiA,* Δ*pflAB,* Δ*adhE* a été réalisée comme précédemment à l'aide de la technique du phage P1 (voir protocole en c)). Le lysat de phage est réalisé avec la souche *E. coli K12 NZN11* Δ*plf :: cam, ldhA ::kana* fourni par Clark D.P. (Bunch P. K., Mat-Jan F. and Clark D. P. (1997) The ldhA gene encoding the fermentative lactate dehydrogenase of Escherichia coli : microbiology, 143, 187-195.). La transduction de la souche MG1655 Δ*tpiA,* Δ*pflAB,* Δ*adhE* est réalisée à l'aide du lysat de phage de la souche *E. coli K12 NZN11* Δ*plf :: cam, ldhA ::kana.* Les transductants sont sélectionnés sur kanamycine et l'insertion de la cassette kanamycine dans le gène *ldhA* est vérifiée à l'aide des oligonucléotides hslJC et ldhAC2.
hsIJC (SEQ ID N° 13) :
gccatcagcaggcttagccg (homologue à la séquence 1439345 à 1439767)
IdhAC2 : (SEQ ID N°14) :
gggtattgtggcatgtttaaccg (homologue à la séquence 1441007 à 1441029)

La souche obtenue est appelée MG1655 Δ*tpiA,* Δ*pflAB,* Δ*adhE, ldhA:: kana*

### g) construction d'une souche modifiée E. coli MG1655Δ gloA::cm :

L'inactivation du gène *gloA* est réalisée comme précédemment en insérant une cassette de résistance à l'antibiotique chloramphénicol tout en délétant la majeure partie des gènes concernés par la technique décrite par Datsenko, K.A. and Wanner, B.L. (2000).

Deux oligonucléotides sont utilisés pour réaliser la délétion : avec :
une région (caractères minuscules) homologue à la séquence (1725861-1725941) du gène *gloA* (séquence 1725861 à 1726268), séquence de référence sur le site http://genolist.pasteur.fr/Colibri/)
une région (caractères majuscules) pour l'amplification de la cassette de résistance au chloramphénicol du plasmide pKD3 (Datsenko, K.A. and Wanner, B.L. (2000)
une région (caractères minuscules) homologue à la séquence (1726188 - 1726268) du gène *gloA* (1725861 -1726268)
une région (caractères majuscules) pour l'amplification de la cassette de résistance au chloramphénicol portée par le plasmide pKD3

Les oligonucléotides GLOADr et GLOADf sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides Nem A C d et Rnt C r .
NemAQd (SEQ ID NO 17) :
gaagtggtcgatgccgggattgaagaatggg (homologue de 1725331 à 1725361)
Rnt Cr (SEQ ID NO18) :
gggttacgtttcagtgaggcgcgttctgcgg (homologue à la séquence de 1726765 à 1726795)

### h) construction d'une souche modifiée E. coli MG1655 ΔtpiA, ΔpflAB, ΔadhE, ldhA :: kana, ΔgloA :

L'inactivation du gène *gloA* dans la souche MG1655 Δ*tpiA,* Δ*pflAB,* Δ*adhE,ldhA :: kana* a été réalisée comme précédemment à l'aide de la technique du phage P1 (voir protocole en c)). Le lysat de phage P1 est effectué sur la souche MG1655 Δ*gloA ::cm,* et la transduction de la souche MG1655 Δ*tpiA,* Δ*pt*/*AB,* Δ*adhE, ldhA :: kana* est réalisée à l'aide de ce lysat. Les transductants résistants au chloramphenicol sont contrôlés à l'aide des oligonucléotides NemAQd et Rnt Cr pour vérifier la mutation du gène *gloA* et aussi à l'aide des oligonucléotides, pflAB1 et pflAB2, cdh et YIIQ, ychCf et adhECr, hsIJC et IdhAC2, afin de vérifier également respectivement la délétion des gènes *pfla* et *B, tpiA , adhE ,* et *IdhA* dans la souche Δ*gloA ::cm*.

Comme précédemment, la cassette de résistance au chloramphénicol est ensuite éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment. La souche obtenue est appelée MG16555 Δ*tpiA,* Δ*pflAB,* Δ*adhE, ldhA :: kana,* Δ*gloA*.

### i) construction d'une souche modifiée E. coli MG1655 Δ aldA :: cm

L'inactivation du gène *aldA* est réalisée comme précédemment en insérant une cassette de résistance à l'antibiotique chloramphénicol tout en délétant la majeure partie des gènes concernés par la technique décrite par Datsenko, K.A. and Wanner, B.L. (2000).

### Deux oligonucléotides sont utilisés pour réaliser la délétion :

avec :
une région (caractères minuscules) homologue à la séquence (1486256 - 1486336) du gène *aldA* (séquence 1486256 à 1487695), séquence de référence sur le site http://genolist.pasteur.fr/Colibri/)
une région (caractères majuscules) pour l'amplification de la cassette de résistance au chloramphénicol du plasmide pKD3 (Datsenko, K.A. and Wanner, B.L. (2000)
une région (caractères minuscules) homologue à la séquence (1487615 - 1487695) du gène *aldA* (1486256 à 1487695)
une région (caractères majuscules) pour l'amplification de la cassette de résistance au chloramphénicol portée par le plasmide pKD3

Les oligonucléotides AldA D r et aldAD f sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides Ydc F C f et gapCCr.
Ydc F C f (SEQ ID NO 21):
tgcagcggcgcacgatggcgacgttccgccg (homologue de 1485722 à 1485752)
gapCCr (SEQ ID NO22) :
cacgatgacgaccattcatgcctatactggc (homologue à la séquence de 1488195 à 1488225)

### h) construction d'une souche modifiée E. coli MG1655 ΔtpiA, ΔpflAB, ΔadhE, IdhA :: kana, ΔgloA , ΔaldA

L'inactivation du gène *aldA* dans la souche MG1655 Δ*tpiA,* Δ*pf*/*AB,* Δ*adhE,* /*dia :: kana,* Δ*gloA* a été réalisée comme précédemment à l'aide de la technique du phage P1 (voir protocole en c)). Le lysat de phage P1 est effectué sur la souche MG1655 Δ*aldA ::cm,* et la transduction de la souche MG1655 Δ*tpiA,* Δ*pf*/*AB,* Δ*adhE, ldhA :: kana,* Δ*gloA* est réalisée à l'aide de ce lysat. Les transductants résistants au chloramphenicol sont contrôlés à l'aide des oligonucléotides Ydc F C f et gapCCr pour vérifier la mutation du gène *aldA* et aussi à l'aide des oligonucléotides, NemAQd et Rnt Cr, pflAB1 et pflAB2, cdh et YIIQ, ychCf et adhECr, hsIJC et ldhAC2, afin de vérifier également respectivement la délétion des gènes *gloA, pflA* et *B, tpiA, adhE* dans la souche Δ*aldA ::cm*.

Comme précédemment, la cassette de résistance au chloramphénicol est ensuite éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment. La souche obtenue est appelée MG16555 Δ *tpiA,* Δ*pflAB,* Δ*adhE, ldhA :: kana,* Δ*gloA,* Δ*aldA*.

### g) construction d'une souche modifiée E. coli MG 1655 ΔaldB ::cm :

L'inactivation du gène *aldB* est réalisée comme précédemment en insérant une cassette de résistance à l'antibiotique chloramphénicol tout en délétant la majeure partie des gènes concernés par la technique décrite par Datsenko, K.A. ; Wanner, B.L. (2000).

Deux oligonucléotides sont utilisés pour réaliser la délétion : avec :
une région (caractères minuscules) homologue à la séquence (3752603-3752683) du gène *aldB* (séquence 3752603 à 3754141), séquence de référence sur le site http://genolist.pasteur.fr/Colibri/)
une région (caractères majuscules) pour l'amplification de la cassette de résistance au chloramphénicol du plasmide pKD3 (Datsenko, K.A. and Wanner, B.L. (2000)
une région (caractères minuscules) homologue à la séquence (3754061-3754141) du gène *aldB* (3752603 à 3754141)
une région (caractères majuscules) pour l'amplification de la cassette de résistance au chloramphénicol portée par le plasmide pKD3.

Les oligonucléotides AldB D r et aldB D f sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides aldB C f et YiaYCr.
aldB C f (SEQ ID NO 25) :
catatttccctcaaagaatataaaaaagaacaattaacgc (homologue à la séquence de 3752057 à 3752095)
YiaYCr (SEQ ID NO26) :
tatgttcatgcgatggcgcaccagctgggcg (homologue à la séquence de 3754644 à 3754674)

### h) construction d'une souche modifiée E. coli MG1655 ΔtpiA, ΔpflAB, ΔadhE, ldhA :: kana, ΔgloA, ΔaldA, ΔaldB

L'inactivation du gène *aldB* dans la souche MG1655 Δ*tpiA,* Δ*pflAB,* Δ*adhE, ldhA :: kana,* Δ*gloA, aldA* a été réalisée comme précédemment à l'aide de la technique du phage P1 (voir protocole en c)). Le lysat de phage P1 est effectué sur la souche MG1655 Δ*aldB ::cm,* et la transduction de la souche MG1655 Δ*tpiA,* Δ*pflAB,* Δ*adhE,* Δ*ldhA :: kana,* Δ*gloA,* Δ*aldA* est réalisée à l'aide de ce lysat. Les transductants résistants au chloramphenicol sont contrôlés à l'aide des oligonucléotides aldB C f et YiaYCr pour vérifier la mutation du gène *aldB* et aussi à l'aide des oligonucléotides, NemAQd et Rnt Cr, pflAB1 et pflAB2, cdh et YIIQ, ychCf et adhECr, hsIJC et ldhAC2, Ydc F C f et gapCCr, afin de vérifier également respectivement la délétion des gènes *gloA, pfla* et *B, tpiA, adhE, aldA* dans la souche Δ*aldB ::cm*.

Comme précédemment, la cassette de résistance au chloramphénicol est ensuite éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment. La souche obtenue est appelée MG1655 Δ*tpiA,* Δ*pflAB,* Δ*adhE, ldhA :: kana,* Δ*gloA,* Δ*aldA,* Δ*aldB*.

### Exemple 2 : Culture et évolution de la souche modifiée E. coli MG1655 Δ tpiA, ΔpflAB, ΔadhE, ldhA :: kana, ΔgloA, ΔaldA, ΔaldB en chemostat :

Pour optimiser la production de 1,2-propanediol à partir de glucose par la souche *E. coli* MG 16555 Δ *tpiA,* Δ*ptlAB,* Δ*adhE ldhA :: kana,* Δ*gloA,* Δ*aldA,* Δ*aldB*, une culture de la souche en chemostat, dans un milieu de culture minimum, supplémenté en nitrate de sodium et en extrait de levure, pendant plusieurs semaines, est réalisée en condition d'anaérobiose.

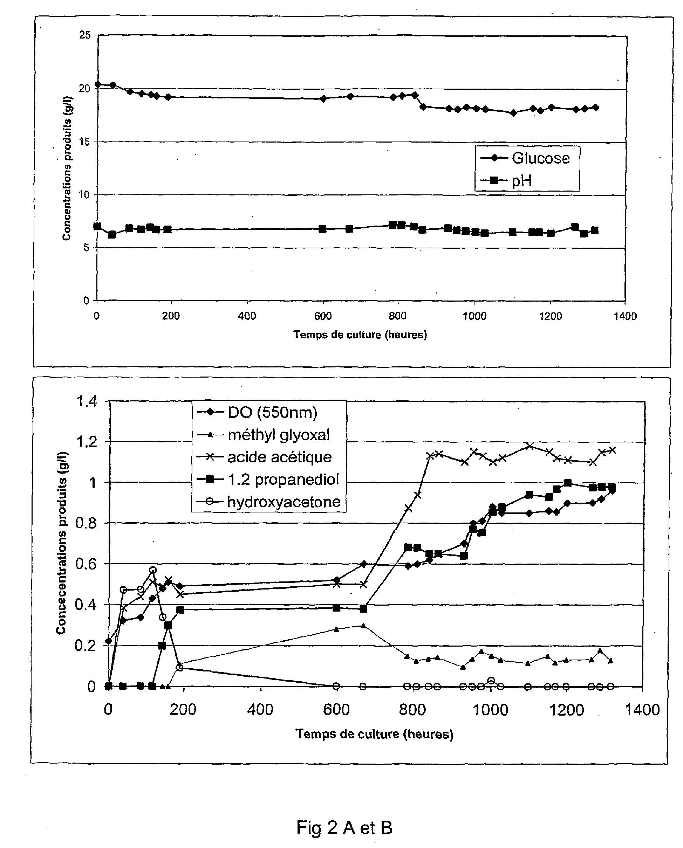

Au début de la culture, la concentration initiale en glucose dans le bidon d'alimentation de la culture est de 20 g/l, le taux de dilution est de 0,04h⁻¹ et un flux d'azote continu est maintenu pour réaliser les conditions d'anérobiose. La concentration cellulaire et les productions en 1,2-propanediol et acétate sont faibles. Après plusieurs semaines de culture, la croissance et les concentrations en produits augmentent, un régime permanent est atteint caractérisé par une concentration en glucose résiduel et des concentrations en produits constantes (figure 2).

### Exemple 3 : Caractérisation d'un complexe pyruvate déshydrogénase évolué peu sensible au NADH :

L'évolution du complexe pyruvate déshydrogènase (PDHc) vers un PDHc peu sensible au NADH est mis en évidence à la fois par un dosage de l'activité de l'enzyme évoluée *in vitro,* et, par comparaison de la séquence d'un des gènes (*lpd*) codant pour la lipoamide déshydrogénase du PDHc évolué avec celle du gène du PDHc natif.
a) dosage de l'activité enzymatique du complexe pyruvate déshydrogènase :

Le dosage de l'activité enzymatique du PDHc *in vitro* de la souche *E. coli** MG1655 Δ *tpiA,* Δ*pf*/*AB,* Δ*adhE, ldhA :: kana,* Δ*gloA,* Δ*aldA,* Δ*aldB* est effectué selon le protocole décrit par Schwartz et Reed (Schwartz E. R. and Reed L. J. (1970) Regulation of the activity of the pyruvate dehydrogenase complex of Escherichia coli, Biochemistry, 6,1434-1439).

Un volume de 100 ml de culture cellulaire est prélevé du chemostat dans des fioles préalablement dégazées et rentrées sous une hotte anaérobie. Les cellules sont centrifugées 10 minutes à 6000 rpm. Le culot est mis en suspension dans environ 100 ml de tampon phosphate de potassium 50 mM, pH7.9, 0.5 mM thiamine pyrophosphate, puis centrifugées de nouveau 10 minutes à 6000 rpm. Le lavage est effectué une deuxième fois dans les mêmes conditions. Le culot cellulaire est mis en suspension dans 800µl de tampon. La suspension cellulaire est désintégrée au moyen d'un appareil à ultrasons en 4 cycles de traitement (30 secondes à 30%), entrecoupés de 2 minutes de repos dans de la glace. Les débris cellulaires sont éliminés par centrifugation 5 minutes à 13400 rpm, le surnageant est l'extrait acellulaire brut. Les sels présents dans l'extrait acellulaire, susceptibles d'interférer dans le dosage enzymatique, sont éliminés par passage de l'extrait à travers une colonne PD10 équilibrée avec du tampon phosphate de potassium pH 7,9, 0,5mM de thiamine pyrophosphate. L'extrait est élué avec 3,5ml du même tampon que précédemment. L'éluat récupéré est l'extrait acellulaire brut.

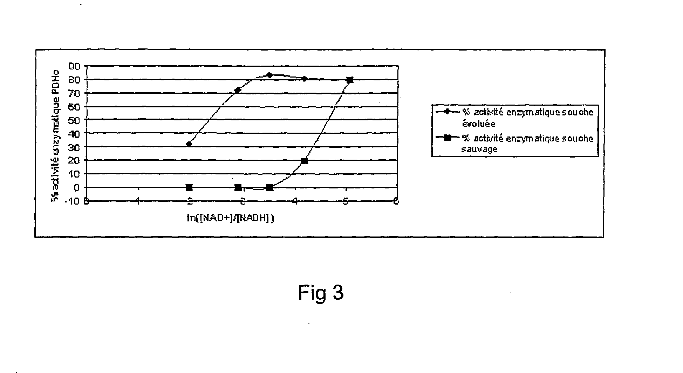

L'activité enzymatique de l'extrait acellulaire brut est mesurée dans un premier temps en absence de NADH, puis dans un deuxième temps en présence de concentrations croissantes de NADH de 0.14 mM à 2.7 mM. Les résultats obtenus sont comparés avec ceux présentés dans la littérature pour la souche *d'E. coli* sauvage figure n° 3 (Snoep J. L., De Graef M. R., Westphal A. H., De Kok A. Teixeira de Mattos M. J. and Neijssel O. M. (1993) Differences in sensitivity to NADH of purified pyruvate dehydrogenase complexes of Enterococcus faecalis, Lactococcus lactis, Azotobacter vinelandii and Escherichia coli : implications for their activity in vivo, FEMS microbiology letters, 114,279-284)).

Les résultats obtenus indiquent que le PDHc de la souche modifiée *E. coli* MG1655 Δ *tpiA,* Δ*pf*/*AB,* Δ*adhE, ldhA :: kana,* Δ*g*/*oA,* Δ*a*/*dA,* Δ*aldB* évoluée est moins sensible au NADH que la souche sauvage *d'E. coli.* Pour un rapport [NAD+]/[NADH] ≅ 33, une inhibition totale de l'activité du PDHc de la souche sauvage est observée, alors que 80% de l'activité du PDHc évolué est mesurée.
b) Détermination de la séquence du gène *lpd* codant pour la lipoamide déshydrogénase du complexe pyruvate déshydrogénase de la souche MG1655 Δ*tpiA,* Δ*pflAB,* Δ*adhE, ldhA :: kana,* Δ*gloA,* Δ*aldA,* Δ*aldB* évoluée :
   L'ADN chromosomique de la souche *E. coli**MG1655 Δ *tpiA,* Δ*pflAB,* Δ*adhE, ldhA :: kana ,* Δ*gloA,* Δ*aldA,* Δ*aldB* est extrait à partir de 1 ml d'une culture de nuit en LB. Après centrifugation, les cellules sont lavées avec de l'eau stérile puis éclatées par choc thermique 5 minutes à 94°C. L'ADN chromosomique est récupéré dans le surnageant après centrifugation. Le gène /*pd* (séquence 127912 à 129336) codant pour la lipoamide déshydrogénase (E3) du complexe pyruvate déshydrogènase est amplifié par PCR à l'aide des deux oligonucléotides suivants :
   AceEf (SEQ ID N°27)
      cgcgtgatcgacggtgctgatggtgcccg (homologue à la séquence 127504 à 127532)
   YacH r (SEQ IDN°28)
      aagttcaggagagccgccc (homologue à la séquence 127513 à 129531)

Un produit PCR de 2000 paires de bases correspondant au gène *lpd* est obtenu et séquencé. Les résultats obtenus montrent la présence d'une mutation ponctuelle entraînant le remplacement de l'alanine 55 par une valine.

### Exemple 4 : La voie de conversion du méthyl glyoxal en 1,2-propanediol de la souche modifiée E. coli MG1655 Δ tpiA, Δpf/AB, ΔadhE, ldhA :: kana, ΔgloA, Δa/dA, ΔaldB évoluée n'implique pas la glycérol déshydrogénase :

Afin de montrer que l'amélioration des performances de la souche modifiée *E. coli* MG1655 Δ *tpiA,* Δ*pflAB,* Δ*adhE, ldhA :: kana,* Δ*gloA,* Δ*aldA,* Δ*aldB* évoluée n'est pas liée à une évolution de la glycérol déshydrogénase codée par le gène *gldA,* une souche *E. coli* MG1655 Δ *tpiA,* Δ*pf*/*AB,* Δ*adhE, ldhA:: kana,* Δ*gloA,* Δ*aldA,* Δ*aldB* évoluée chez laquelle le gène *gldA* est délété a été construite.
a) construction d'une souche modifiée MG1655 Δ*gldA ::cm :*
   L'inactivation du gène *gldA* est réalisée comme indiqué dans l'exemple 1 en insérant une cassette de résistance à l'antibiotique chloramphénicol tout en délétant la majeure partie des gènes concernés par la technique décrite par Datsenko, K.A. ; Wanner, B.L. (2000).

Deux oligonucléotides sont utilisés pour réaliser la délétion : avec:
une région (caractères minuscules) homologue à la séquence (4135512 à 4135592) du gène *gldA* (séquence 4135512 à 4136615), séquence de référence sur le site http://genolist.pasteur.fr/Colibri/)
une région (caractères majuscules) pour l'amplification de la cassette de résistance au chloramphénicol du plasmide pKD3 (Datsenko, K.A. and Wanner, B.L. (2000)
une région (caractères minuscules) homologue à la séquence (4136535-4136615) du gène *gldA* (4135512 à 4136615)
une région (caractères majuscules) pour l'amplification de la cassette de résistance au chloramphénicol portée par le plasmide pKD3

Les oligonucléotides gldA D r et gldA D f sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides YijF D et TalCr.
YijF D (SEQ ID NO 31) :
gcctggatttgtaccacggttggtggaacggcggg (homologue à la séquence de 4135140 à 4135174)
TalCr (SEQ ID NO 32) :
cacgcatattccccattgccgggg (homologue à la séquence de 4137216 à 4137239)

Un produit PCR de 2100 pb est obtenu pour le gène sauvage comme pour le gène délété et remplacé par le gène de résistance au chloramphénicol. Aussi, les produits PCR obtenus sont ensuite digérés par l'enzyme de restriction Sall. Deux fragments d'environ 1000 pb sont obtenus pour le produit PCR sauvage alors que le produit PCR contenant le gène de résistance au chloramphénicol n'est pas digéré.

### b) construction d'une souche modifiée MG1655 Δ tpiA, ΔpflAB, ΔadhE, ldhA :: kana, ΔgloA, ΔaldA, ΔaldB ΔgldA ::cm évoluée

L'inactivation du gène *gldA* dans la souche MG1655 Δ*tpiA,* Δ*pf*/*AB,* Δ*adhE, ldhA:: kana, ,* Δ*gloA,* Δ *aldA* Δ*aldB* évoluée a été réalisée comme dans l'exemple 1 à l'aide de la technique du phage P1 (voir protocole en c)). Le lysat de phage P1 est effectué sur la souche MG1655 Δ*gldA ::cm,* et la transduction de la souche MG1655 Δ*tpiA,* Δ*pflAB,* Δ*adhE,* /*dhA:: kana,* Δ*gloA,* Δ*a*/*dA* est réalisée à l'aide de ce lysat. Les transductants résistants au chloramphenicol sont contrôlés à l'aide des oligonucléotides YijF D et TalCr pour vérifier la mutation du gène *gldA.*

### c) Culture des souches modifiées E. coli *MG 1655 Δ tpiA, ΔpflAB, ΔadhE, ldhA :: kana, ΔgloA, ΔaldA, ΔaldB ΔgldA ::cm évoluée et E. coli *MG 1655 Δ tpiA, ΔpflAB, ΔadhE, ldhA :: kana, ΔgloA, Δalda, ΔaldB évoluée :

Les deux souches modifiées *E. coli* *MG 1655 Δ *tpiA,* Δ*pfiAB,* Δ*adhE, ldhA :: kana,* Δ*gloA,* Δ*aldA,* Δ*aldB* Δ*gldA :: cm* évoluée *et E. coli* *MG 1655 Δ *tpiA,* Δ*pflAB,* Δ*adhE, ldhA:: kana ,* Δ*gloA,* Δ*aldA,* Δ*aldB* évoluée sont cultivées à pH non régulé dans un milieu de culture minimum supplémenté en nitrate de sodium et en extrait de levure avec une concentration initiale en glucose de 20g/l en condition d'anaérobiose pendant 10 jours. Le profil de produits de fermentation obtenu montre que la délétion du gène *gldA* n'entraine pas une diminution de la production de 1.2 propanediol (tableau 1).

**Tableau 1: Comparaison des concentrations en substrat et produits de fermentation après 10 jours de culture des souches modifiées E. coli *MG 1655 Δ tpiA, ΔpflAB, ΔadhE, ldhA :: kana, ΔgloA, ΔaldA, ΔaldB Δg/dA ::cm évoluée et E. coli *MG 1655 Δ tpiA, ΔpflAB, ΔadhE, ldhA :: kana, ΔgloA, ΔaldA, Δa/dB évoluée :**

| Souches | Densité optique | Glucose consommé (g/l) | Méthyl glyoxal (g/l) | Acide acétique (g/l) | 1.2 propanediol (g/l) |
|---|---|---|---|---|---|
| *E. coli* *MG 1655 Δ*tpiA,* Δ*pflAB,* Δ*adhE, dhA :: kana,* Δ*gloA,* Δ*aldA,* Δ*aldB,* Δ*gldA ::cm* | 1,5 | 7,3 | 1,2 | 2,1 | 1,7 |
| *E. coli* *MG 1655 Δ*tpiA,* Δ*pflAB,* Δ*adhE, dhA :: kana,* Δ*gloA,* Δ*aldA,* Δ*aldB,* | 1,9 | 9,6 | 1,4 | 1,9 | 1,2 |

### Exemple 5 : Amélioration du rendement de conversion du glucose en 1.2 propanediol par délétion du gène edd codant pour la 6-phospho-gluconate déshydratase dans la souche E. coli *MG 1655 Δ tpiA, ΔpflAB, ΔadhE, ldhA :: kana, ΔgloA, ΔaldA, ΔaldB évoluée :

### a) construction d'une souche modifiée MG1655 Δedd::cm :

L'inactivation du gène *edd* est réalisée comme indiqué dans l'exemple 1 en insérant une cassette de résistance à l'antibiotique chloramphénicol tout en délétant la majeure partie des gènes concernés par la technique décrite par Datsenko, K.A. ; Wanner, B.L. (2000).

Deux oligonucléotides sont utilisés pour réaliser la délétion :
edd D f de 100 bases (SEQ ID N°33)
   ttaaaaagtgatacaggttgcgccctgttcggcaccggacagtttttcacgcaaggcgctgaataattcacgtcctgt tcGTGTAGGCTGGAGCTGCTTCG
   avec :
   une région (caractères minuscules) homologue à la séquence (1930817à 4) du gène *edd* (séquence 1930817 à 1932628), séquence de référence sur le site http://genolist.pasteur.fr/Colibri/)
   une région (caractères majuscules) pour l'amplification de la cassette de résistance au chloramphénicol du plasmide pKD3 (Datsenko, K.A. and Wanner, B.L. (2000)
   une région (caractères minuscules) homologue à la séquence (1932548-1932628) du gène edd(séquence 1930817 à 1932628)
   une région (caractères majuscules) pour l'amplification de la cassette de résistance au chloramphénicol portée par le plasmide pKD3

Les oligonucléotides edd D r et edd D f sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides eda d et zwf r :
Eda d (SEQ ID NO 35) :
   CCCCGGAATCAGAGGAATAGTCCC (homologue à la séquence de 1930439 à 1930462)
Zwf r (SEQ ID NO 36) :
   GGGTAGACTCCATTACTGAGGCGTGGGCG (homologue à la séquence de 1932968 à 1932996)

### b) construction d'une souche modifiée MG1655 Δ tpiA, ΔpflAB, ΔadhE, ldhA :: kana, ΔgloA, ΔaldA, ΔaldB Δedd ::cm évoluée

L'inactivation du gène *edd* dans la souche MG1655 Δ*tpiA,* Δ*pflAB,* Δ*adhE, ldhA :: kana, ,* Δ*gloA,* Δ *aldA,* Δ*aldB* évoluée a été réalisée comme dans l'exemple 1 à l'aide de la technique du phage P1 (voir protocole en c)). Le lysat de phage P1 est effectué sur la souche MG1655 Δ*edd ::cm,* et la transduction de la souche MG1655 Δ*tpiA,* Δ*pflAB,* Δ*adhE, ldhA :: kana,* Δ*gloA,* Δ*aldA,* Δ*aldB* est réalisée à l'aide de ce lysat. Les transductants résistants au chloramphenicol sont contrôlés à l'aide des oligonucléotides eda d et zwf r pour vérifier la mutation du gène *edd.*

### c) Culture des souches modifiées E. coli *MG 1655 Δ tpiA, Δpf/AB, ΔadhE, ldhA :: kana , ΔgloA, ΔaldA, ΔaldB, Δedd ::cm évoluée et E. coli *MG 16555 Δ tpiA, ΔpflAB, ΔadhE, ldhA :: kana, ΔgloA, ΔaldA, ΔaldB évoluée :

Les deux souches modifiées *E. coli* *MG 1655 Δ *tpiA,* Δ*pf*/*AB,* Δ*adhE, ldhA :: kana,* Δ*gloA,* Δ*aldA,* Δ*aldB,* Δ*edd ::cm* évoluée *et E. coli* *MG 1655 Δ *tpiA,* Δ*pflAB,* Δ*adhE, ldhA :: kana,* Δ*gloA,* Δ*aldA,* Δ*a*/*dB* évoluée sont cultivées à pH non régulé dans un milieu de culture minimum supplémenté en nitrate de sodium et en extrait de levure avec une concentration initiale en glucose de 20g/l en condition d'anaérobiose pendant 10 jours. Le profil de produits de fermentation obtenu montre que la délétion du gène *edd* entraine une augmentation du rendement de conversion du glucose en 1,2-propanediol de 0,13 g/g à 0,35 g/g (tableau 2). La délétion du gène *edd* dans la souche *E. coli* *MG 1655 Δ *tpiA,* Δ*pf*/*AB,* Δ*adhE, ldhA :: kana,* Δ*gloA,* Δ*aldA,* Δ*a*/*dB* évoluée permet donc d'améliorer les performances de la souche.

**Tableau 2 : Comparaison des concentrations en substrat et produits de fermentation après 10 jours de culture des souches modifiées E. coli *MG 1655 Δ tpiA, ΔpflAB, ΔadhE, ldhA :: kana, Δg/oA, ΔaldA, ΔaldB Δedd ::cm évoluée et E. coli *MG 1655 Δ tpiA, ΔpflAB, ΔadhE, ldhA :: kana, ΔgloA, ΔaldA, Δa/dB évoluée :**

| Souches | Densité optique | Glucose consommé (g/l) | Méthyl glyoxal (g/l) | Acide acétique (g/l) | 1,2-propanediol (g/l) | Y 1.2 pdiol/glucose (g/g) |
|---|---|---|---|---|---|---|
| *E. coli* * Δ*tpiA,* Δ*pflAB,* Δ*adhE, dhA :: kana ,*Δ*gloA,* Δ*aldA,* Δ*aldB,* Δ*edd ::cm* | 1.2 | 5 | 0,2 | 1,5 | 1,8 | 0,35 |
| *E. coli* * Δ*tpiA,* Δ*pflAB,* Δ*adhE, dhA :: kana,* Δ*g*/*oA,* Δ*aldA,* Δ*aldB,* | 1,9 | 9,6 | 1,4 | 1,9 | 1,2 | 0,13 |

### Exemple 6 : Construction d'une souche modifiée E. Coli MG1655 Δ tpiA, ΔpflAB, ΔadhE, ldhA :: kana, ΔgloA, ΔaldA, ΔaldB, Δedd ::cm (pSOS95T) capable de produire du 1,2-propanediol et de l'acétone :

Le plasmide appelé pSOS95T est un vecteur navette d'expression pour *E. coli*/*C. acetobutylicum* portant l'opéron acétone de *Clostridum acetobutylicum* constitué de quatre gènes *adc, ctfAB, thl* codant respectivement pour l'acétoacétate carboxylase, la coenzyme A transférase et la thiolase sous la dépendance du promoteur thiolase. Ces trois enzymes catalysent la transformation de l'acétyl-CoA et de l'acétate en acétone et dioxyde de carbone. Le plasmide pSOS95T a été obtenu par insertion dans le plasmide pSOS95 (Gene bank numéro d'accession AY187686) du gène *thl* de *C. acetobutylicum* codant pour la thiolase, au site BamH1 situé entre le promoteur thiolase et le gène *ctfA.* Le plasmide pSOS95T est introduit dans la souche *E. coli** MG1655 Δ *tpiA,* Δ*pflAB,* Δ*adhE, ldhA :: kana,* Δ*gloA,* Δ*a*/*dA,* Δ*aldB,* Δ*edd:: cm* évoluée par électroporation.

Des cellules électrocompétentes de la souche *E. coli** MG1655 Δ *tpiA,* Δ*pflAB,* Δ*adhE, ldhA :: kana,* Δ*g*/*oA,* Δ*a*/*dA,* Δ*a*/*dB,* Δ*edd :: cm* sont préparées à partir d'une culture de nuit de la souche en LB. Une culture de 10 ml LB en erlenmeyer est ensemencée au 100^{ième} par la culture de nuit et incubée à 37°C. Lorsque la densité optique à 550 nm de la culture atteint une valeur comprise entre 0.4 et 0.6, 1 ml de culture est centrifugée. Les cellules sont lavées avec de l'eau, puis avec une solution de glycérol à 10%, avec d'être resuspendues dans 0.05 ml de d'une solution de glycérol à 10 %. Les cellules sont électroporées immédiatement (25 µF, 200 Ω, 2.5KV) (Gene pulser, Biorad) avec 5 µl de la préparation plasmidique pSOS95T (Qiagen, Hilden germany). Après 1 heure d'expression phénotypique en milieu SOC (Sambrook J., Fristch E. F. and Maniatis T. (1989) Molecular cloning : a laboratory manual, 2 nd ed Cold Spring Harbor Laboratory, cold Spring Harbor, N.Y.) à 37°C, les transformants sont sélectionnés sur milieu gélosé avec de la carbenicilline 100 µg/ml à 37°C.

Les transformants sont remis en culture liquide en présence de carbénicilline pendant une nuit pour réaliser une extraction d'ADN plasmidique (Qiagen, Hilden Germany) afin de contrôler la présence du plasmide pSOS95T et de vérifier par digestion enzymatique qu'il est correct.

### Exemple 7 : Culture de la souche modifiée E. coli *MG1655 Δ tpiA, ΔpflAB, ΔadhE, ldhA :: kana, ΔgloA, Δa/dA, ΔaldB, Δedd :: cm (pSOS95T) évoluée capable de produire du 1,2-propanediol et de l'acétone :

La souche modifiée *E. coli** MG1655 Δ *tpiA,* Δ*pflAB,* Δ*adhE, ldhA :: kana ,* Δ*gloA,* Δ*aldA,* Δ*aldB* Δ*edd :: cm (pSOS95T)* évoluée est cultivée à pH non régulé dans un milieu de culture minimum supplémenté en nitrate de sodium et en extrait de levure avec une concentration initiale en glucose de 20g/l en condition d'anaérobiose (tableau 3). Le dosage des produits de fermentation montre que la souche *E. Coli** MG1655 Δ *tpiA,* Δ*pflAB,* Δ*adhE, ldhA :: kana,* Δ*gloA,* Δ*a*/*dA*, Δ*a*/*dB* Δ*edd :: cm (pSOS95T)* évoluée produit un mélange de 1,2-propanediol, d'acétate et d'acétone.

**Tableau 3 : Comparaison des concentrations en substrat et produits de fermentation après 10 jours de culture des souches modifiées E. coli *MG 1655 Δ tpiA, ΔpflAB, ΔadhE, ldhA :: kana , Δg/oA, ΔaldA, ΔaldB, Δedd ::cm évoluée et E. coli *MG 1655 Δ tpiA, ΔpflAB, ΔadhE, ldhA :: kana, ΔgloA, ΔaldA, ΔaldB, Δedd :: cm évoluée :**

| Souches | Densité optique | Glucose consommé (g/l) | Méthyl glyoxal (g/l) | Acide acétique (g/l) | 1,2-propanediol (g/l) | Acétone (g/l) |
|---|---|---|---|---|---|---|
| *E. coli* * Δ*tpiA,* Δ*pflAB,* Δ*adhE, dhA :: kana,* Δ*gloA,* Δ*aldA,* Δ*aldB,* Δ*edd :: cm (pSOS95T)* | 1,4 | 4,8 | 0,3 | 1,3 | 1,6 | 0,1 |
| *E. coli* * Δ*tpiA,* Δp*flAB,* Δ*adhE, ldhA :: kana,* Δ*gloA,* Δ*aldA,* Δ*aldB,* Δ*edd :: cm* | 1,2 | 5 | 0,2 | 1,5 | 1,8 | / |

### REFERENCES

✔ Altaras N. E. and Cameron D. (1999) Metabolic engineering of a 1,2-propanediol pathway in Escherichia coli : Appl. Env. Microb., 65, 1180-1185.
✔ Altaras N. E. and Cameron D. C. (2000)Enhanced production of ( R ) 1,2-propanediol by metabolically engineered Escherichia coli : Biotechnol. Prog. 16, 940-946
✔ Altaras NE, Etzel MR and Cameron DC. (2001) Conversion of sugars to 1,2-propanediol by Thermoanaerobacterium thermosaccharolyticum HG-8 : Biotechnol. Prog. 17, 52-56
✔ Bunch P. K., Mat-Jan F. and Clark D. P. (1997) The ldhA gene encoding the fermentative lactate dehydrogenase of Escherichia coli : microbiology, 143, 187-195.
✔ Cameron D. C., Altaras N. E., Hoffman M. L. and Shaw A. J. (1998) Metabolic engineering of propanediol pathways : Biotechnol. Prog., 14, 116-125.
✔ Cameron D. C., Shaw A. J. and Altaras N. E. (1998) Microbial production of 1,2-propanediol from sugar WO 98/37204
✔ Cameron D. C., Shaw A. J. and Altaras N. E. (2000) Microbial production of 1,2-propanediol from sugar US 6 087 140
✔ Cameron D. C., Shaw A. J. and Altaras N. E. (2001) Microbial production of 1,2-propanediol from sugar US 6 303 352
✔ Carrier T A and Keasling J. D. (1999) Library of synthetic 5' secondary structures to manipulate mRNA stability in Escherichia coli, Biotechnol. prog., 15, 58-64
✔ Cheperanov P. P. and Wackernagel W. (1995) Gene disruption in Escherichia coli : TCR and KmR cassettes with option of Flp-catalyzed excision of the antibiotic-resistance determinant, gene, 158, 9-14
✔ Datsenko, K.A. and Wanner, B.L. (2000) One-step inactivation of chromosomal genes in Escherichia coli K-12 using PCR products. Proc. Natl. Acad. Sci. USA 97 : 6640-6645
✔ Sambrook J., Fristch E. F. and Maniatis T. (1989) Molecular cloning : a laboratory manual, 2 nd ed Cold Spring Harbor Laboratory, cold Spring Harbor, N.Y.
✔ Schwartz E. R. and Reed L. J. (1970) Regulation of the activity of the pyruvate dehydrogenase complex of Escherichia coli, Biochemistry, 6,1434-1439
✔ Snoep J. L., De Graef M. R., Westphal A. H., De Kok A. Teixeira de Mattos M. J. and Neijssel O. M. (1993) Differences in sensitivity to NADH of purified pyruvate dehydrogenase complexes of Enterococcus faecalis, Lactococcus lactis, Azotobacter vlnelandii and Escherichia coli : implications for their activity in vivo, FEMS microbiology letters, 114, 279-284)).
✔ Tran Din K. and Gottschalk G. (1985) Formation of D(-)-1,2-propanediol and D(-)-lactate from glucose by Clostridium sphenoides under phosphate limitation : Arch. Microbiol. 142, 87-92.

## Revendications

1. Procédé de préparation d' une souche de microorganismes évolués pour la production de 1,2-propanediol par métabolisme d' une source de carbone simple, à partir d' une souche initiale comprenant une délétion du gène *tpiA* et une délétion d' au moins un gène codant pour une enzyme impliquée dans la conversion du méthyl glyoxal (propanal) en lactate
ledit procédé comprenant les étapes suivantes de :
• cultiver ladite souche initiale dans un milieu de culture approprié, comprenant une source de carbone simple, en appliquant des taux de dilution croissants de telle sorte de ne conserver dans le milieu de culture que les microorganismes ayant un taux de croissance égal ou supérieur au taux de dilution imposé , afin de faire évoluer dans ladite souche initiale un ou plusieurs gènes impliqués dans la voie de biosynthèse du DHAP en méthylglyoxal puis en 1,2-propanediol vers des gènes évolués ayant une activité « 1,2-propanediol synthase » améliorée,
• sélectionner et isoler la ou les souches de microorganismes évolués ayant une activité « 1,2-propanediol synthase » améliorée.

2. Procédé selon la revendication 1, **caractérisé en ce que** le gène impliqué dans la conversion du méthyl glyoxal en lactate est choisi parmi *gloA, aldA* et *aldB.*

3. Procédé selon la revendication 2, **caractérisé en ce que** la souche initiale comprend la délétion des gènes *aldA* et/ou *aldB.*

4. Procédé selon l'une des revendications 1 ou 2, **caractérisé en ce que** la souche initiale comprend la délétion des gènes *gloA, aldA, aldB* et *tpiA.*

5. Procédé selon l'une des revendications 1 à 4, **caractérisé en ce que** dans la souche initiale, les voies naturelles de fermentation du glucose qui sont consommatrices de NADH sont supprimées.

6. Procédé selon l'une des revendications 1 à 5, **caractérisé en ce que** la souche initiale comprend la délétion des gènes *adhE et* /*dha.*

7. Procédé selon l'une des revendications 1 à 6, **caractérisé en ce que** la souche initiale comprend également au moins un gène codant pour un complexe pyruvate deshydrogenase.

8. Procédé selon la revendication 7, **caractérisé en ce que** le gène *lpd* codant pour la lipoamide dehydrogenase du complexe pyruvate déshydrogénase porte une mutation ponctuelle entrainant une modification de l' alanine 55 en valine.

9. Procédé selon l'une des revendications 7 ou 8, **caractérisé en ce que** le complexe pyruvate déshydrogénase est endogène.

10. Procédé selon l'une des revendications 1 à 9, **caractérisé en ce que** la souche initiale comprend la délétion des gènes *pflA et pflB.*

11. Procédé selon l'une des revendications 1 à 10, **caractérisé en ce que** la souche initiale comprend également une délétion du gène *edd.*

12. Procédé selon l'une des revendications 1 à 11, **caractérisé en ce que** la souche initiale comprend une délétion des gènes *gloA, aldA, aldB, tpiA, adhE, ldhA, pflA, pflB,* et *edd.*

13. Procédé selon l' une des revendications 1 à 12, **caractérisé en ce que** l'on introduit dans la souche évoluée un ou plusieurs gènes hétérologues codant pour une ou plusieurs enzymes impliquées dans la conversion de l' acétyl-CoA et de l' acétate en acétone, ces enzymes étant choisies parmi l' acétoacétate carboxylase, la coenzyme A transferase et la thiolase.

14. Procédé selon la revendication 13, **caractérisé en ce que** le ou les gènes hétérologues codant pour une ou plusieurs enzymes impliquées dans la conversion de l' acétyl-CoA et de l' acétate proviennent de *Clostridium acetobutylicum.*

15. Procédé selon l'une des revendications 13 ou 14, **caractérisé en ce** l' on cultive sous pression de sélection dans un milieu de culture approprié comprenant une source de carbone simple une souche évoluée modifiée obtenue selon l'une des revendications 13 ou 14 afin de faire évoluer dans ladite souche évoluée modifiée un ou plusieurs gènes impliqués dans la conversion de l' acétyl-CoA et de l'acétate en acétone vers une activité « acétone synthase » améliorée, puis on sélectionne et on isole les microorganismes évolués de deuxième génération ayant une activité « 1,2-propanediol synthase » améliorée et une activité « acétone synthase » améliorée.

16. Procédé selon l'une des revendications 1, à 15, **caractérisé en ce que** la souche est choisie parmi les bactéries, les levures et les champignons.

17. Procédé selon la revendication 16, **caractérisé en ce que** la souche est choisie parmi une souche d' *Escherichia,* en particulier *E. coli,* et de *Corynebacterium,* en particulier *C. glutamicum.*

18. Souche initiale telle que définie selon l'une des revendications 3 à 6, 8, 10, 11 ou 12.

19. Souche initiale selon la revendication 18, **caractérisée en ce qu'** elle est choisie parmi les bactéries, les levures et les champignons.

20. Souche initiale selon la revendication 19, **caractérisée en ce qu'** elle est choisie parmi une souche d' *Escherichia,* en particulier *E. coli,* et de *Corynebacterium,* en particulier *C. glutamicum.*

21. Souche évoluée obtenue par le procédé selon l'une des revendications 1 à 17 dans laquelle le gène *lpd* porte une mutation ponctuelle entrainant une modification de l' alanine 55 en valine.

22. Procédé de préparation de 1,2-propanediol dans lequel on cultive une souche évoluée selon la revendication 21 dans un milieu de culture approprié comprenant une source simple de carbone, puis on récupère le 1,2-propanediol produit.

23. Procédé selon la revendication 22, **caractérisé en ce que** l' on récupère du 1,2-propanediol et de l' acétone.

24. Procédé selon l' une des revendications 22 ou 23, **caractérisé en ce que** le 1,2-propanediol et/ou l' acétone sont purifiés.

## Claims

1. A method for the preparation of a strain of evolved micro-organisms for the production of 1,2-propanediol by the metabolism of a simple carbon source, from an initial strain comprising a deletion of the *tpiA* gene and a deletion of at least one gene coding for an enzyme involved in the conversion of methylglyoxal (propanal) into lactate,
said method comprising the following steps:
■ growing said initial strain in an appropriate culture medium containing a simple carbon source, by applying increasing dilution rates so as to only retain in the culture medium microorganisms which have a growth rate equal to or greater than the imposed dilution, in order to cause evolution, in said initial strain, of one or more genes involved in the biosynthesis pathway from DHAP to methylglyoxal and then to 1,2-propanediol towards evolved genes having an improved "1,2-propanediol synthase" activity,
■ selecting and isolating the evolved strain(s) of micro-organisms having an improved "1,2-propanediol synthase activity".

2. The method according to claim 1, **characterized in that** the gene involved in the conversion of methylglyoxal into lactate is *gloA, aldA* or *aldB.*

3. The method according to claim 2, **characterized in that** the initial strain comprises a deletion of the genes *aldA* and/or *aldB.*

4. The method according to any of claims 1 or 2, **characterized in that** the initial strain comprises a deletion of the genes *gloA, aldA*, *aldB* and *tpiA.*

5. The method according to any of claims 1 to 4, **characterized in that** in the initial strain, the natural routes for fermentation of glucose which consume NADH are suppressed.

6. The method according to any of claims 1 to 5, **characterized in that** the initial strain comprises deletion of the genes *adhE* and *ldha.*

7. The method according to any of claims 1 to 6, **characterized in that** the initial strain also comprises at least one gene coding for pyruvate dehydrogenase complex.

8. The method according to claim 7, **characterized in that** the gene *lpd* coding for the lipoamide dehydrogenase of the pyruvate dehydrogenase complex bears a point mutation whereby alanine 55 is replaced by valine.

9. The method according to any of claims 7 or 8, **characterized in that** the pyruvate dehydrogenase complex is endogenous.

10. The method according to any of claims 1 to 9, **characterized in that** the initial strain comprises a deletion of the genes *pflA* and *pflB.*

11. The method according to any of claims 1 to 10, **characterized in that** the initial strain also comprises a deletion of the gene *edd*.

12. The method according to any of claims 1 to 11, **characterized in that** the initial strain comprises deletion of the genes *gloA, aldA, aldB, tpiA, adhE, ldhA, pflA, pflB* and *edd*.

13. The method according to any of claims 1 to 12, **characterized in that** one or more heterologous genes which code for one or more enzymes involved in the conversion of acetyl-CoA and acetate into acetone are introduced into the evolved strain, these enzymes being selected from acetoacetate carboxylase, coenzyme A transferase and thiolase.

14. The method according to claim 13, **characterized in that** the heterologous gene(s) coding for one or more enzymes involved in the conversion of acetyl-CoA and acetate into acetone are from *Clostridium acetobutylicum.*

15. The method according to any of claims 13 or 14, **characterized in that** an evolved modified strain obtained according to any of claims 13 or 14 is grown under selection pressure in an appropriate growth medium containing a simple carbon source in order to cause, in said evolved modified strain, the evolution of one or more genes involved in the conversion of acetyl-CoA and acetate into acetone towards an improved "acetone synthase" activity, and then the evolved second generation micro-organisms having improved "1,2-propanediol synthase" activity and an improved "acetone synthase" activity are then selected and isolated.

16. The method according to any of claims 1 to 15, **characterized in that** the strain is selected from bacteria, yeasts or fungi.

17. The method according to claim 16, **characterized in that** the strain is selected from a strain of *Escherichia,* in particular *E. coli*, and *Corynebacterium,* in particular *C. glutamicum.*

18. An initial strain as defined according to any of claims 3 to 6, 8, 10, 11 or 12.

19. The initial strain according to claim 18, **characterized in that** it is selected from bacteria, yeasts or fungi.

20. The initial strain according to claim 19, **characterized in that** it is selected from a strain of *Escherichia,* in particular *E. coli*, and *Corynebacterium,* in particular *C.glutamicum*

21. An evolved strain obtained according to the method of any of claims 1 to 17, wherein the gene *lpd* bears a point mutation whereby alanine 55 is replaced by valine.

22. A method for preparing 1,2-propanediol wherein an evolved strain according to claim 21 is grown in an appropriate growth medium containing a simple carbon source, and the produced 1,2-propanediol is recovered.

23. The method according to claim 22, **characterized in that** 1,2-propanediol and acetone are recovered.

24. The method according to any of claims 22 or 23, **characterized in that** the 1,2-propanediol and/or acetone are purified.

## Patentansprüche

1. Verfahren zur Herstellung eines Stamms von Mikroorganismen, die sich dahingehend fortentwickelt haben, dass sie 1,2-Propandiol durch Verstoffwechslung einer einfachen Kohlenstoffquelle produzieren, wobei von einem Ursprungsstamm ausgegangen wird, der eine Deletion des Gens *tpiA* und eine Deletion mindestens eines Gens, das für ein Enzym codiert, welches an der Umwandlung von Methylglyoxal (Propanal) in Lactat beteiligt ist, umfasst, wobei das Verfahren die folgenden Schritte umfasst:
• Kultivieren des Ursprungsstamms in einem geeigneten Kulturmedium, das eine einfache Kohlenstoffquelle umfasst, wobei zunehmende Verdünnungsgrade derart zur Anwendung kommen, dass nur solche Mikroorganismen in dem Kulturmedium verbleiben, deren Wachstumsrate größer oder gleich dem auferlegten Verdünnungsgrad ist, damit in dem Ursprungsstamm ein oder mehrere Gene, die am Biosyntheseweg von DHAP zu Methylglyoxal und anschließend zu 1,2-Propandiol beteiligt sind, sich zu Genen fortentwickeln, die eine verbesserte "1,2-Propandiol-Synthase"-Aktivität haben,
• Selektieren und Isolieren des oder der Stämme von fortentwickelten Mikroorganismen, die eine verbesserte "1,2-Propandiol-Synthase"-Aktivität haben.

2. Verfahren nach Anspruch 1, **dadurch gekennzeichnet, dass** das Gen, welches an der Umwandlung von Methylglyoxal in Lactat beteiligt ist, aus *gloA, aldA* und *aldB* gewählt ist.

3. Verfahren nach Anspruch 2, **dadurch gekennzeichnet, dass** der Ursprungsstamm die Deletion der Gene *aldA* und/oder *aldB* umfasst.

4. Verfahren nach einem der Ansprüche 1 oder 2, **dadurch gekennzeichnet, dass** der Ursprungsstamm die Deletion der Gene *gloA, aldA, aldB* und *tpiA* umfasst.

5. Verfahren nach einem der Ansprüche 1 bis 4, **dadurch gekennzeichnet, dass** diejenigen natürlichen Wege der Glucosefermentation, bei welchen NADH verbraucht wird, beim Ursprungsstamm ausgeschaltet sind.

6. Verfahren nach einem der Ansprüche 1 bis 5, **dadurch gekennzeichnet, dass** der Ursprungsstamm die Deletion der Gene *adhE* und *ldha* umfasst.

7. Verfahren nach einem der Ansprüche 1 bis 6, **dadurch gekennzeichnet, dass** der Ursprungsstamm weiterhin mindestens ein Gen umfasst, das für einen Pyruvat-Dehydrogenase-Komplex codiert.

8. Verfahren nach Anspruch 7, **dadurch gekennzeichnet, dass** das Gen *lpd,* welches für die Lipoamid-Dehydrogenase des Pyruvat-Dehydrogenase-Komplexes codiert, eine Punktmutation aufweist, die zu einer Änderung des Alanins 55 in Valin führt.

9. Verfahren nach einem der Ansprüche 7 oder 8, **dadurch gekennzeichnet, dass** der Pyruvat-Dehydrogenase-Komplex endogen ist.

10. Verfahren nach einem der Ansprüche 1 bis 9, **dadurch gekennzeichnet, dass** der Ursprungsstamm die Deletion der Gene *pflA* und *pflB* umfasst.

11. Verfahren nach einem der Ansprüche 1 bis 10, **dadurch gekennzeichnet, dass** der Ursprungsstamm weiterhin eine Deletion des Gens *edd* umfasst.

12. Verfahren nach einem der Ansprüche 1 oder 11, **dadurch gekennzeichnet, dass** der Ursprungsstamm eine Deletion der Gene *gloA, aldA, aldB, tpiA, adhE, ldhA, pflA, pflB* und *edd* umfasst.

13. Verfahren nach einem der Ansprüche 1 bis 12, **dadurch gekennzeichnet, dass** in den fortentwickelten Stamm ein oder mehrere heterologe Gene eingeführt werden, die für ein oder mehrere Enzyme codieren, welche an der Umwandlung von Acetyl-CoA und von Acetat in Aceton beteiligt sind, wobei diese Enzyme aus der Acetoacetat-Carboxylase, der Coenzym-A-Transferase und der Thiolase gewählt sind.

14. Verfahren nach Anspruch 13, **dadurch gekennzeichnet, dass** das oder die heterologen Gene, die für ein oder mehrere Enzyme codieren, welche an der Umwandlung von Acetyl-CoA und von Acetat beteiligt sind, von *Clostridium acetobutylicum* stammen.

15. Verfahren nach einem der Ansprüche 13 oder 14, **dadurch gekennzeichnet, dass** ein veränderter, fortentwickelter Stamm, der gemäß einem der Ansprüche 13 oder 14 erhalten wurde, in einem geeigneten Kulturmedium, das eine einfache Kohlenstoffquelle enthält, unter Selektionsdruck kultiviert wird, damit sich in dem veränderten, fortentwickelten Stamm ein oder mehrere Gene, die an der Umwandlung von Acetyl-CoA und von Acetat in Aceton beteiligt sind, dahingehend fortentwickeln, dass sie eine verbesserte "Aceton-Synthase"-Aktivität aufweisen, woraufhin die fortentwickelten Mikroorganismen der zweiten Generation, die eine verbesserte "1,2-Propandiol-Synthase"-Aktivität und eine verbesserte "Aceton-Synthase"-Aktivität haben, selektiert und isoliert werden.

16. Verfahren nach einem der Ansprüche 1 bis 15, **dadurch gekennzeichnet, dass** der Stamm aus den Bakterien, den Hefen und den Pilzen gewählt ist.

17. Verfahren nach Anspruch 16, **dadurch gekennzeichnet, dass** der Stamm aus einem Stamm von Escherichia, insbesondere E. *coli*, und von *Corynebacterium*, insbesondere *C. glutamicum*, gewählt ist.

18. Ursprungsstamm gemäß der Begriffsbestimmung in einem der Ansprüche 3 bis 6, 8, 10, 11 oder 12.

19. Ursprungsstamm nach Anspruch 18, **dadurch gekennzeichnet, dass** er aus den Bakterien, den Hefen und den Pilzen gewählt ist.

20. Ursprungsstamm nach Anspruch 19, **dadurch gekennzeichnet, dass** er aus einem Stamm von *Escherichia*, insbesondere *E. coli*, und von *Corynebacterium*, insbesondere *C. glutamicum*, gewählt ist.

21. Fortentwickelter Stamm, welcher mittels eines Verfahrens nach einem der Ansprüche 1 bis 17 erhalten wurde und bei welchem das Gen *1pd* eine Punktmutation aufweist, die zu einer Änderung des Alanins 55 in Valin führt.

22. Verfahren zur Herstellung von 1,2-Propandiol, wobei ein fortentwickelter Stamm nach Anspruch 21 in einem geeigneten Kulturmedium, das eine einfache Kohlenstoffquelle umfasst, kultiviert wird, woraufhin das produzierte 1,2-Propandiol gewonnen wird.

23. Verfahren nach Anspruch 22, **dadurch gekennzeichnet, dass** 1,2-Propandiol und Aceton gewonnen werden.

24. Verfahren nach einem der Ansprüche 22 oder 23, **dadurch gekennzeichnet, dass** das 1,2-Propandiol und/oder Aceton aufgereinigt wird/werden.
